# EUROPEAN PATENT SPECIFICATION

(11) **EP 2 790 587 B1**
(45) Date of publication and mention of the grant of the patent: **11.04.2018**
(21) Application number: 12863641.2
(22) Date of filing: 18.12.2012
(51) Int. Cl.: A61B 8/00, A61B 8/08

(54) **THREE DIMENSIONAL MAPPING DISPLAY SYSTEM FOR DIAGNOSTIC ULTRASOUND MACHINES**
ANZEIGESYSTEM MIT DREIDIMENSIONALER KARTIERUNG FÜR DIAGNOSTISCHE ULTRASCHALLMASCHINEN
SYSTÈME D'AFFICHAGE À MAPPAGE TRIDIMENSIONNEL POUR MACHINES DE DIAGNOSTIC ULTRASONORES

(30) Priority: 18.12.2011 US 201161577029 P
(43) Date of publication of application: 22.10.2014
(73) Proprietor: METRITRACK, INC., Hillside, IL 60162 (US)
(72) Inventor: CALUSER, Calin, Glen Ellyn, IL 60137 (US); LIN, Fang, Chicago, IL 60640 (US); ANDREI, Silviu, Glen Ellyn, IL 60137 (US)
(74) Representative: Schäfer, Matthias W.
(86) International application number: PCT/US2012/070423
(87) International publication number: WO 2013/101562

(56) References cited:
- EP-A1- 1 623 674
- WO-A1-98/39669
- US-A1- 2007 055 150
- US-A1- 2007 167 801
- US-A1- 2009 124 906
- US-A1- 2009 124 906
- US-A1- 2011 028 844
- US-A1- 2011 182 493

## Description

### I. Technical Field

The present invention relates to diagnostic ultrasound technology and, more particularly, to a three dimensional mapping display ("TDMD") diagnostic ultrasound system in which ultrasound probe position registration is automated, the position of each pixel in the ultrasound image in reference to preselected anatomical references is calculated, and specified information is stored on command. Moreover, the system, during real time ultrasound scanning enables the ultrasound probe position and orientation to be continuously displayed over a body or body part diagram, thereby facilitating the storage of information. The TDMD can then record multiple ultrasound free hand two-dimensional (also, "2D") frames in a video sequence (clip) or cine loop wherein multiple 2D frames of one or more video sequences corresponding to a scanned volume can be reconstructed in three-dimensional (also,"3D") volume images corresponding to the scanned region, using known 3D reconstruction algorithms.

### II. Background of the Invention

Ultrasound is an important imaging modality for medical diagnostic purposes and as a guidance tool for diagnostic or therapeutic procedures, like soft tissue needle biopsy, tumor ablation, etc. Ultrasound can be used over the entire human body and has certain advantages over other modalities, including, among others: the ability to locate and characterize medical problems; lower cost compared to modalities such as MRI and CT; real time operation; and, the lack of ionizing radiation with the known associated health risks.

Ultrasound imaging systems transmit sound waves of very high frequency (e.g., 1 MHz to 20 MHz) into the patient's body and the echoes scattered from structures in the patient's body are processed to create and display images and information related to these structures.

Ultrasound imaging can be applied to various regions or organs in the body. For example, a breast ultrasound procedure involves the placement of an ultrasound transducer over a region of interest of the breast, with the radiologist or other medical professional (the "user") viewing a real-time ultrasound image output on a display. The ultrasound machine monitor usually displays relevant text and/or graphical information next to the ultrasound image for simultaneous viewing by the user. The user can freeze a displayed image with medical findings of interest, and the corresponding image can be printed on a printer or stored in digital format.

2D free hand ultrasound imaging, the most common technique used today, represents a slice through the region of interest. 3D ultrasound scanning is available; however, it is usually used in conjunction with 2D scanning techniques. Currently, most diagnostic studies are performed using 2D scanning technique.

The vast majority of ultrasound guided biopsies and other invasive ultrasound guided invasive procedures done by free hand and other more automated modes use the ultrasound machine 2D display mode. Therefore, it is desirable to have a fast and accurate way to find the target during such invasive procedures.

It is important to accurately store positional annotations for later evaluation, since this is essential for final interpretation, diagnosis, and treatment. As digital storage and communication of medical information replace hard copy based storage and communication technologies, the accurate and consistent annotation of ultrasound and other medical images is critical. Correlation of ultrasound images with images of the same body region obtained with other modalities (MRI, CT, mammograms, PET, etc.) becomes increasingly important for medical diagnostic and therapeutic purposes. As a result, precise positional registration of the targets is important.

This importance is illustrated by noting that finding a small tumor can save a patient's life. The smaller the tumor is before treatment, the higher the probability of long term patient survival or cure; however, a small tumor is difficult to find in a patient's body and differentiate from other structures or artifacts in the same region. Many times a suspicious small finding can coexist in the same region with multiple benign findings (cysts, solid benign nodules, etc.) with similar appearance, which may create confusion during a follow µp exam and may lead to missing the suspicious lesion. As imaging diagnostic devices provide ever greater detail and sub-millimeter resolution, accurate position registration and mapping of lesions is becoming increasingly important in order to take advantage of the increased capabilities.

Ultrasound procedures are highly dependent on the device user's experience and training. Position recording of certain findings is important, especially for the small targets and /or multiple targets. Most frequently, an ultrasound user will hold the ultrasound transducer in one hand and use the other hand to operate the ultrasound machine controls. It is desirable to obtain the instant recording of target coordinates seen in the ultrasound image in relation to the anatomical reference (for example, a nipple) and the simultaneous recording of the transducer position. Currently, the automated recording of the transducer position in real time scanning is limited due to the motion of the pre-selected anatomical reference secondary to body and transducer induced motion. Therefore, it is desirable to continuously update the position of the anatomical references, or landmarks, and apply the correction to the obtained measurements.

The American College of Radiology (ACR) recommends that all ultrasound images be properly labeled. For example, for breast ultrasound images, the findings position, in hourly format, distance from Nipple C and ultrasound probe position and orientation should be displayed with the ultrasound images. Currently, ultrasound findings are manually labeled by an operator, which is time consuming and prone to errors. Manual labeling involves the typing of an approximate position in the organ or part of the body, since an accurate position registration is time consuming and, importantly, difficult for the user.

Document US 2009/0124906 A1 describes a system where the ultrasound transducer position registration is automated. Although multiple ultrasound guidance systems and devices already exist, they do not offer a practical and accurate solution to mapping patient findings in 2D or 3D images in relation to set anatomical reference(s) which is operator independent during a routine examination, with real time correction for the patient's motion. It would be beneficial, therefore, to obtain the accurate position of selected targets in the ultrasound images in relation to set anatomical reference point(s) with the corresponding ultrasound probe position and orientation by selecting the target in the ultrasound image at the time of examination or at a later date in the stored images with attached positional information. The present invention provides such an advance to the art.

### III. Objects and Advantages of the Present Invention

It is an object of the present invention to significantly reduce the time of the examination by eliminating the time consuming manual labeling of images and speeding up the target finding at subsequent examinations.

It is a further object of the present invention to obtain the accurate position of selected targets in ultrasound images in relation to set anatomical reference(s) with the corresponding ultrasound probe position and orientation by selecting the target in the ultrasound image at the time of examination or at a later time or at a later date in the stored images with attached positional information in both 2D or 3D imaging techniques.

It is a further object of the present invention to enhance correlation capability with other diagnostic imaging modalities like CT scans, MRI, mammograms etc.

It is yet a further object of the present invention to eliminate or minimize errors due to inaccurate position labeling and excessive artifacts, therefore reducing the risk of costly lawsuits due to missed diagnosis and decrease the number of callbacks for the patients for repeat examination.

It is yet a further object of the present invention to provide a sensor attaching device to enable accurate sensor placement and adherence and to, further, reduce the chance of operator error.

One advantage, among the many that will be appreciated by those skilled in the arts, is that the present invention provides an easy, uniform, method of communicating the target position among healthcare providers by guiding the ultrasound to a previously recorded target through following the real time display of the ultrasound transducer position in relation to the target coordinates from a previous examination.

### IV. Summary of the Invention

The present invention provides an apparatus and method of use for automated ultrasound probe position registration, calculating the position of each pixel in the ultrasound image in dynamic reference to the selected anatomical references (AR), and storing selected information on demand. The present invention further enables, during real time ultrasound scanning, continuous ultrasound probe position and orientation display, which display be permanently stored in the system's memory at the users command.

The Present invention comprises a hardware/software application and real time commercial 3D position registration system interfaced with an ultrasound machine.

After initial calibration and selection of one or more anatomical references (nipple, umbilicus, skull, etc.), positional information associated with each individually recorded image frame or each image in a cine loop is stored with the corresponding image. Using a pointing device with the system display, spatial numerical coordinates of the selected pixel or region, including the distance from the anatomical reference, depth, angle to the body axis and a graphical representation, are displayed next to the ultrasound image. Also displayed are the real time position of the ultrasound probe and target position in a body diagram or other representation shown with the real time ultrasound image, to help the ultrasound operator during scanning. The data from the positional sensors is used to perform the dynamic coregistration of the real time ultrasound image, first image set, with the breast or other body part representation, the second image set, in the same spatial coordinate frame. The real time ultrasound image can be coregistered with any number of sets of images previously recorded.

Each saved ultrasound image or set of images in a cine loop will have attached positional information corresponding to each pixel in the ultrasound frame and the diagram with the body part with the ultrasound probe position and orientation in reference to the anatomical reference(s) and position of a target pixel(s), if any pixels are selected. In one embodiment the anatomical reference sensor (48) can be applied at the nipple of the breast (C) when the corresponding breast is examined with the ultrasound machine. Other body parts or regions can be recorded with corresponding anatomical reference(s) for example: liver with umbilicus, neck with thyroid cartilage etc. Target pixel selection can be made at the time of the image capture, before saving the image, or at a later time at the review station.

During future examinations, the user is guided to the target by entering the target coordinates obtained at the previous examination, display the target in the body diagram and adjust the probe position in the real time body diagram to overlap the target.

For the accurate automated recording of body targets and probe position related to certain anatomical references, a user continuously obtains positional information from selected anatomical references sensors and the probe positional coordinates are instantly updated

This is achieved by continuously monitoring the preset anatomical references position, which in the preferred embodiment can be achieved with a magnetic sensor placed next to the anatomical reference on the skin. In an alternate embodiment the anatomical reference tracking can be obtained with an overhead tracking system using digital infrared or optical cameras with or without skin markers. In this embodiment, one camera can be used, or two or more cameras can also be used to achieve a three dimensional stereoscopic effect.

The TDMD can also be used to record multiple ultrasound free hand 2D frames in a video sequence (clip) or cine loop, with each frame saved with the positional coordinates as described above. When using the positional information in the multiple 2D frames of one or more video sequences corresponding to a scanned volume, the 2D images can be used to reconstruct 3D volume containing images corresponding to the scanned region, using known 3D reconstruction algorithms. The 3D volume reconstruction can be obtained from the original captured 2D ultrasound images or the segmented or otherwise processed 2D images in a video sequence. This embodiment is well suited for ultrasound breast cancer screening or diagnostic breast ultrasound exams and can also be applied to other regions in the body like, but not restricted to the eye, liver, abdomen, neck, kidneys, etc.

A sensor attaching device may also be employed to assist in the positioning and adherence of the magnetic or other type of positional sensors and to reduce operator error in the placement of the sensors and interference from connecting wire feedback.

There has been outlined, rather broadly, the more important features of the invention in order that the detailed description thereof that follows may be better understood, and in order that the present contribution to the art may be better appreciated. There are, of course, additional features of the invention that will be described hereinafter and that will form the subject matter of the invention.

### V. Brief description of the drawings

**Fig.1** depicts an overview illustration of the inventive apparatus placed in an ultrasound system.
**Fig.2** illustrates the functional block diagram for the inventive device preferred embodiment with a magnetic sensor used for anatomical reference tracking and another magnetic sensor used for ultrasound probe tracking.

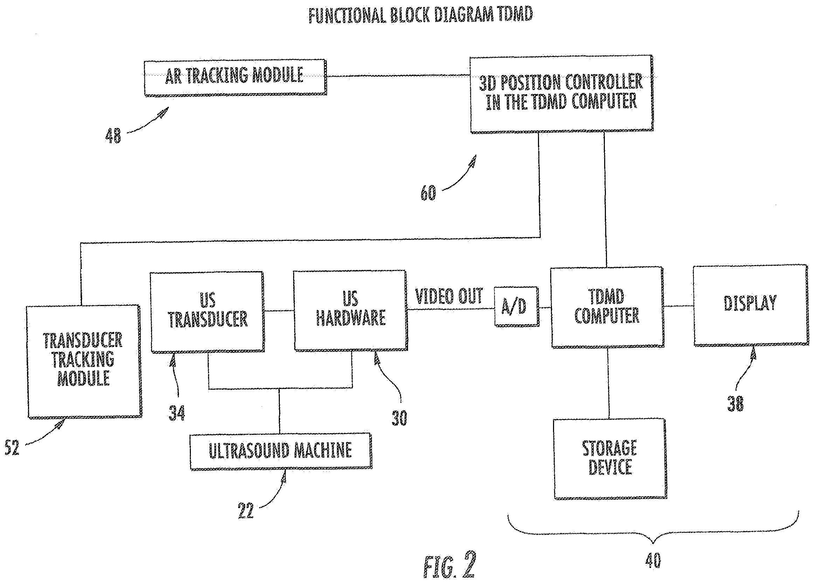

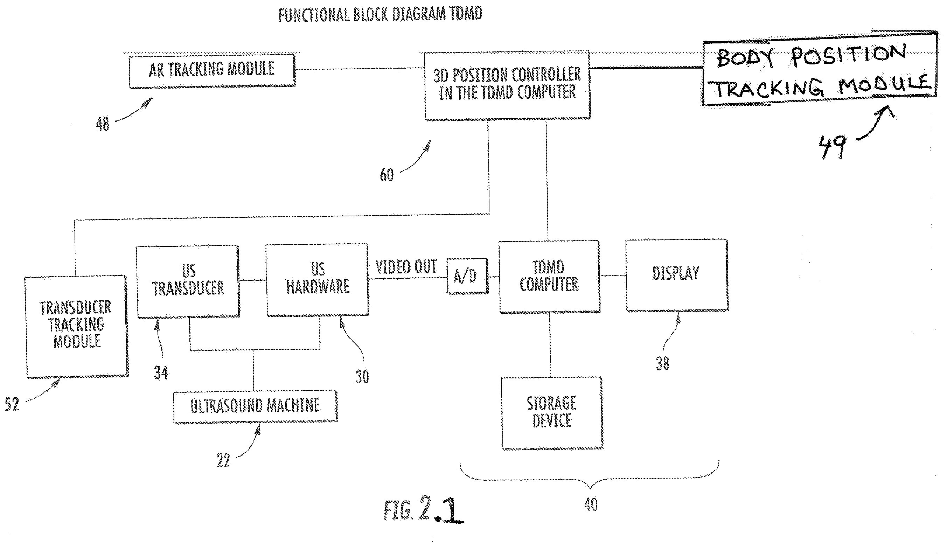

**Fig.2****.****1** illustrates the functional block diagram for the inventive device preferred embodiment with a position sensor used for anatomical reference tracking and another position sensor for body position and orientation tracking.

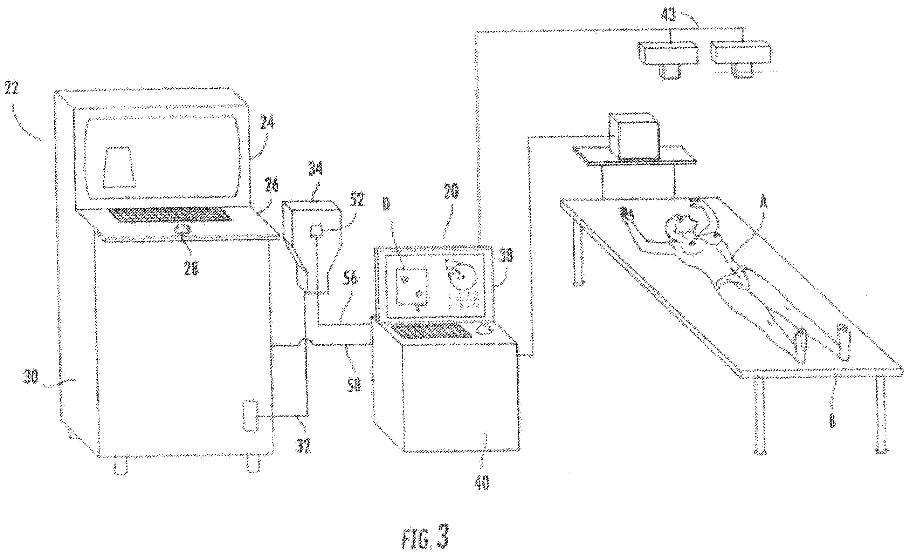

**Fig.3** depicts an alternate embodiment illustrating an overhead infrared or optical anatomical reference tracking system.

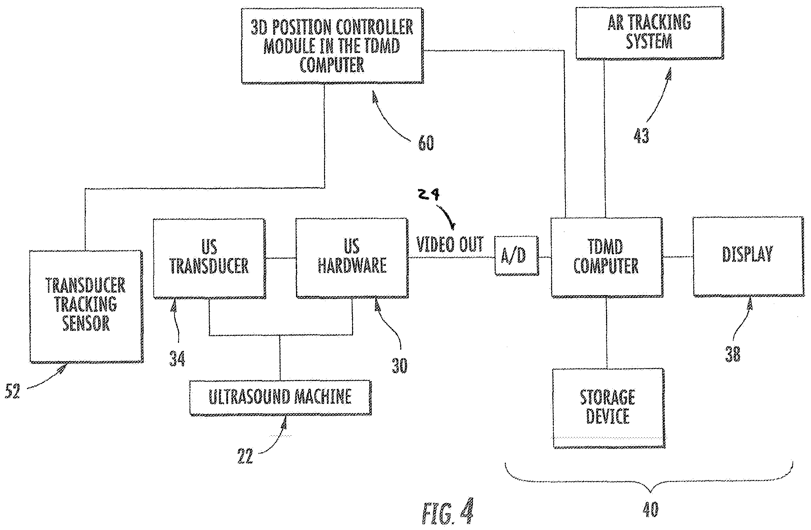

**Fig.4** illustrates the functional block diagram for the inventive device in the alternate embodiment with an overhead infrared or optical anatomical reference tracking system.

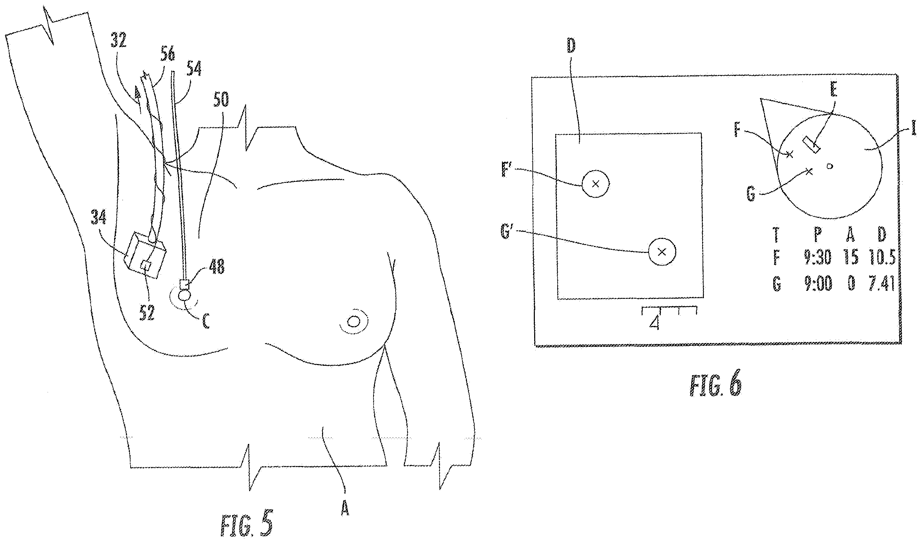

**Fig.5** depicts the inventive apparatus with sensor attached in a breast ultrasound examination
**Fig 6** depicts the image created during a breast examination as illustrated in Figure 5.

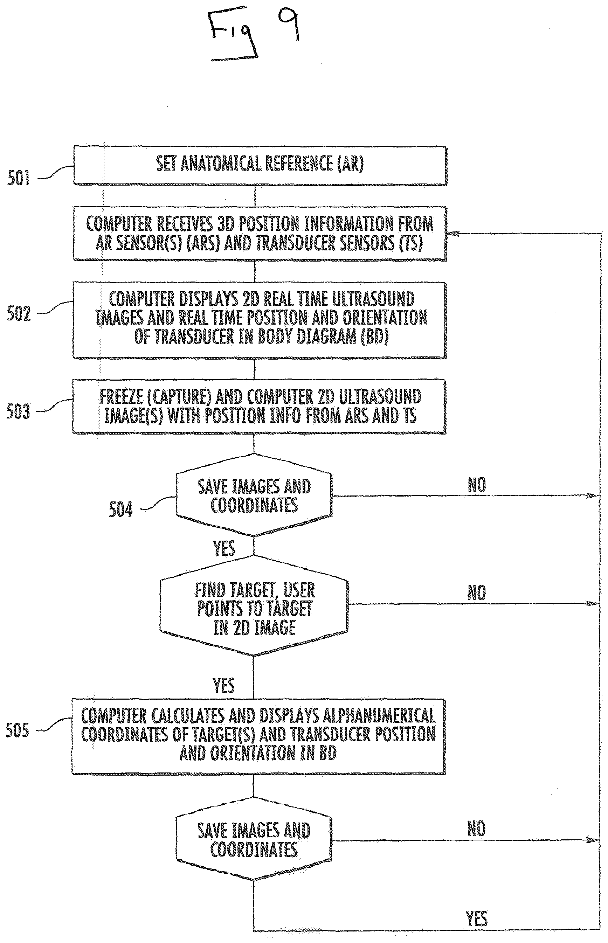

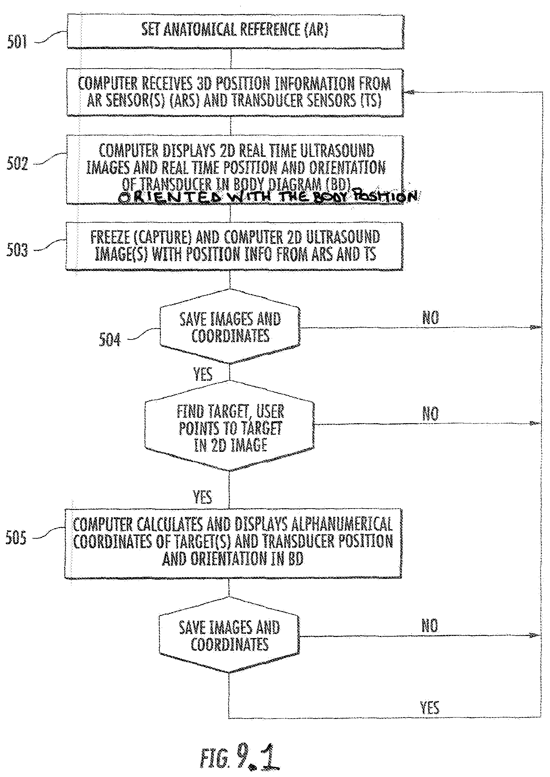

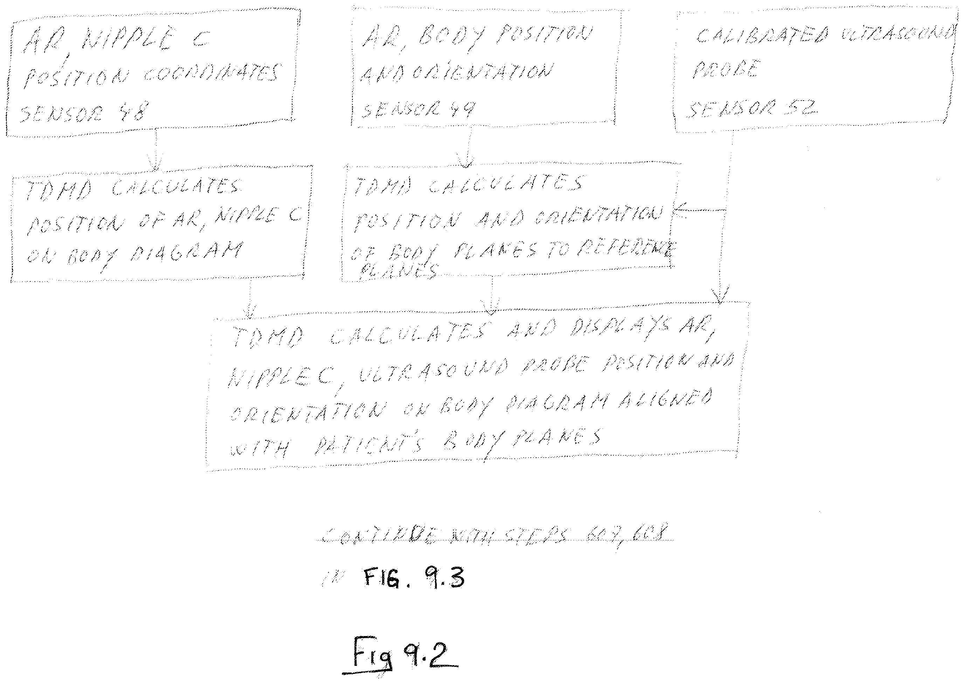

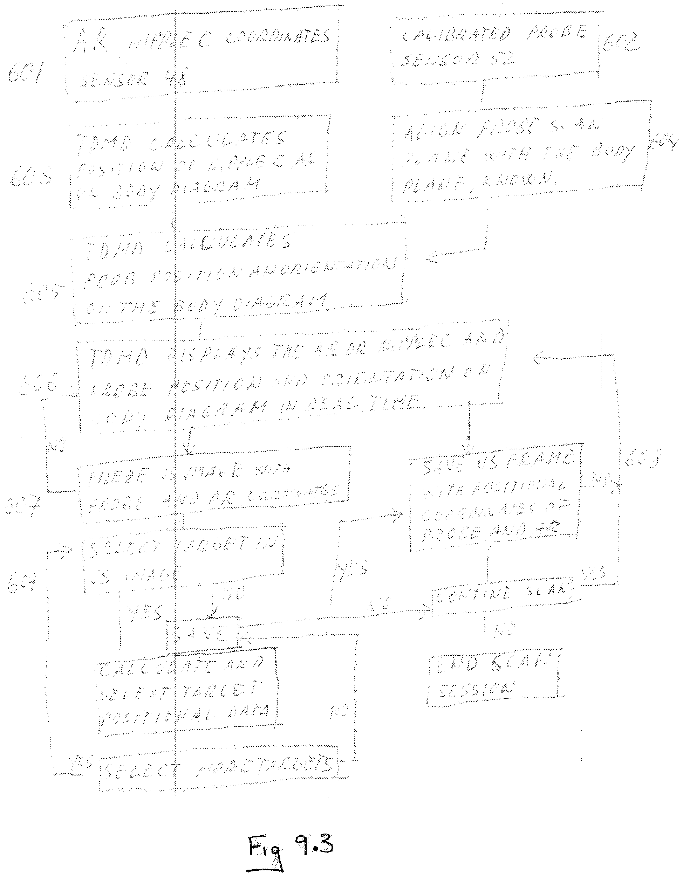

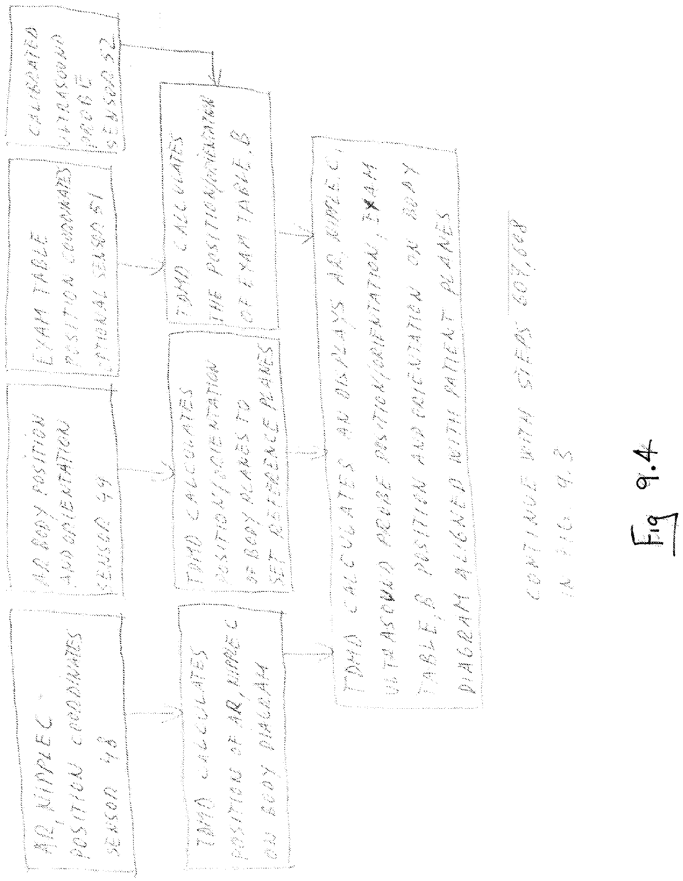

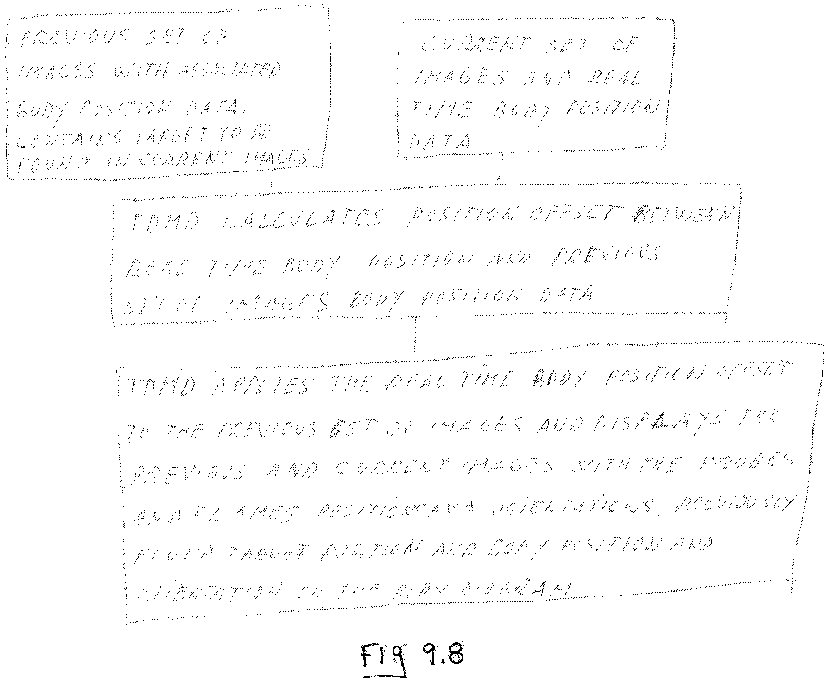

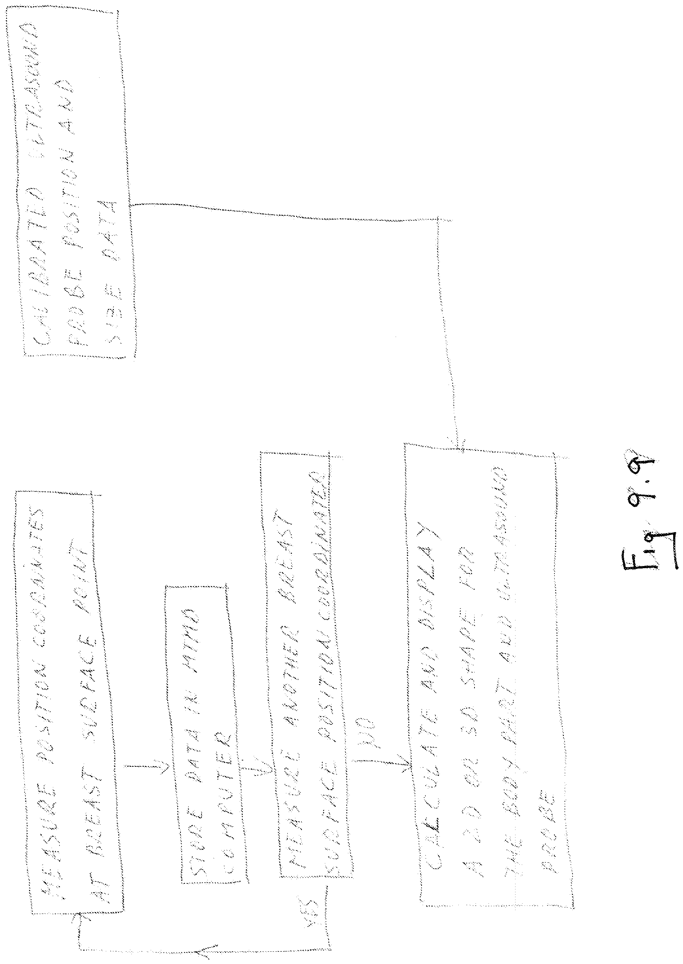

**Fig.9** illustrates the steps needed to measure and record the positional information associated with the diagnostic ultrasound images with a position sensor used for anatomical reference tracking.
**Fig.9****.****1** illustrates the steps needed to measure and record the positional information associated with the diagnostic ultrasound images with a position sensor used for anatomical reference tracking and another position sensor for body position and orientation tracking.
**Fig.9****.****2** illustrates the steps needed to calculate, display and record the positional information associated with the diagnostic ultrasound images with a position sensor used for anatomical reference tracking and another position sensor for body position and orientation tracking.
**Fig.9****.****3** illustrates one method of patient's body planes registration and the steps needed to calculate, display and record the positional information associated with the diagnostic ultrasound images with a position sensor used for anatomical reference tracking and another position sensor for body position and orientation tracking.
**Fig.9****.****4** illustrates the steps needed to calculate, display and record the positional information associated with the diagnostic ultrasound images with a position sensor used for anatomical reference tracking, another position sensor for body position and orientation tracking and exam table position input.
**Fig.9.8** illustrates the steps needed to calculate the positional offset between the body position and orientation in 2 temporally different image sets, apply and display the calculated data over a body diagram.
**Fig.9.9** illustrates the steps needed to calculate and display a realistic same scale representation of a breast with the ultrasound probe and frame position and orientation.

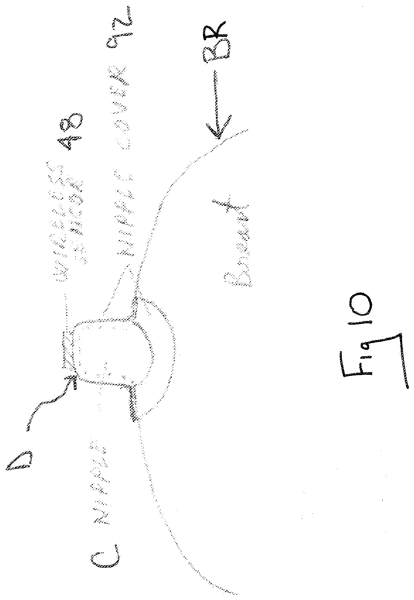

**Fig. 10** illustrates a representative nipple attachment cover with a position sensor or marker.

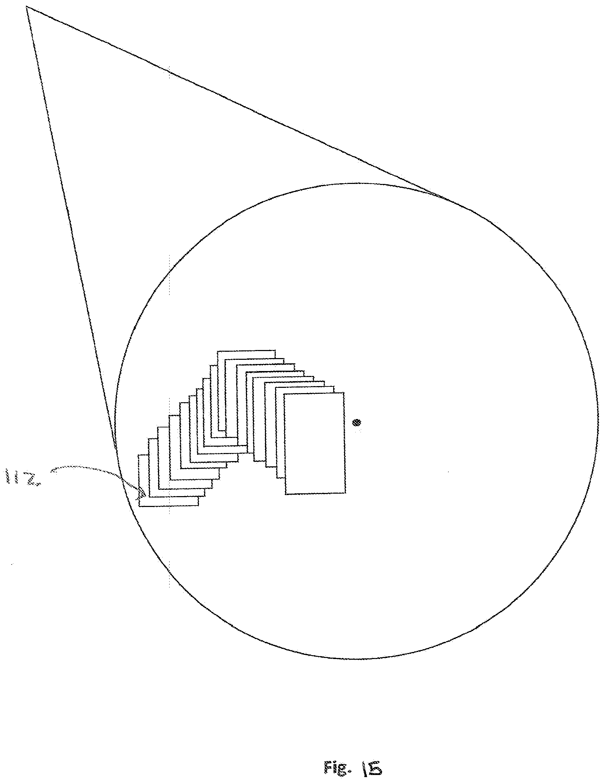

**Fig. 15** depicts a display screen shot illustrating positioning of ultrasound frames in one video clip over a body diagram.

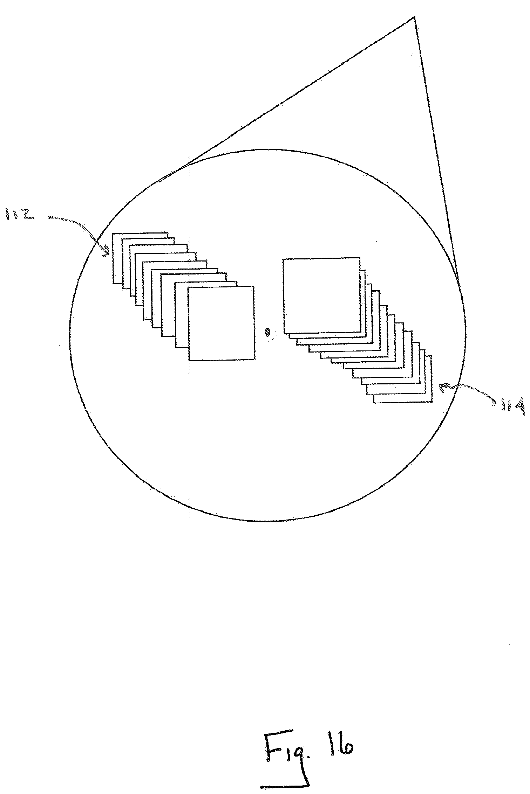

**Fig. 16** depicts a display screen shot illustrating positioning of ultrasound frames in multiple video clips over a body diagram

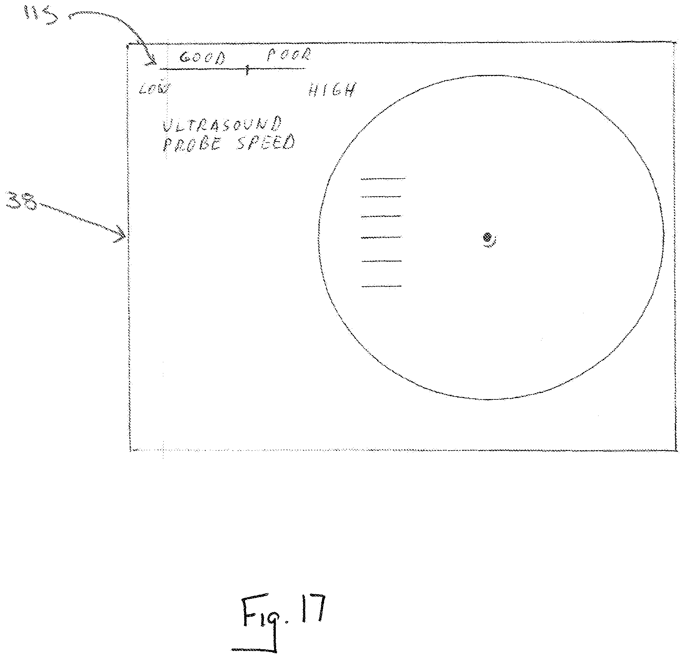

**Fig. 17** depicts a display screen shot illustrating probe speed display with probe and frame positions over a body diagram.

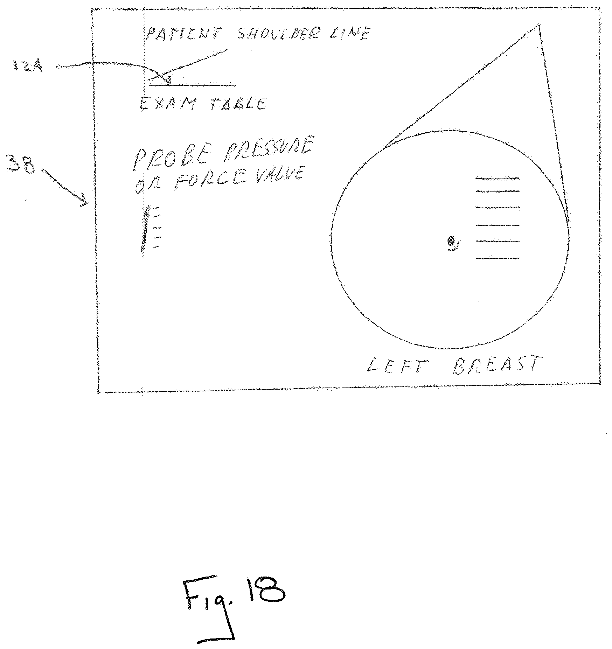

**Fig. 18** depicts a display screen shot illustrating patient position with respect to exam table and corresponding body diagram with ultrasound frames.

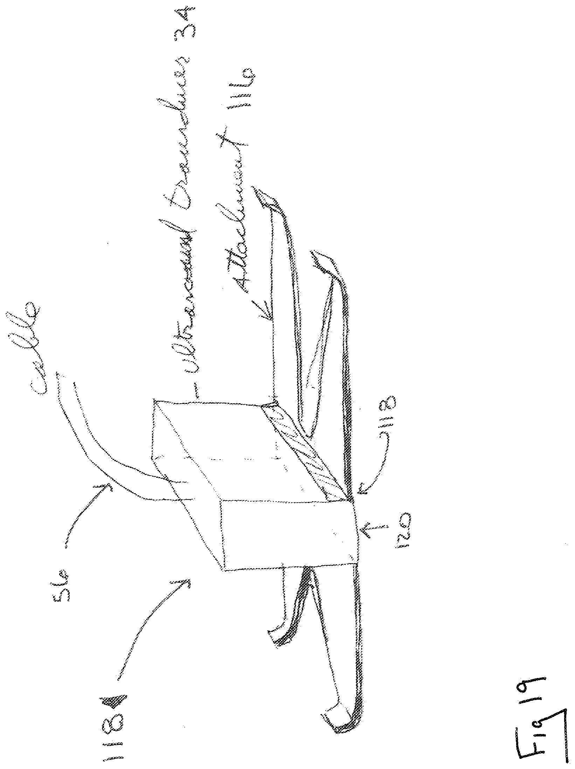

**Fig. 19** depicts an ultrasound probe attachment for breast ultrasound.

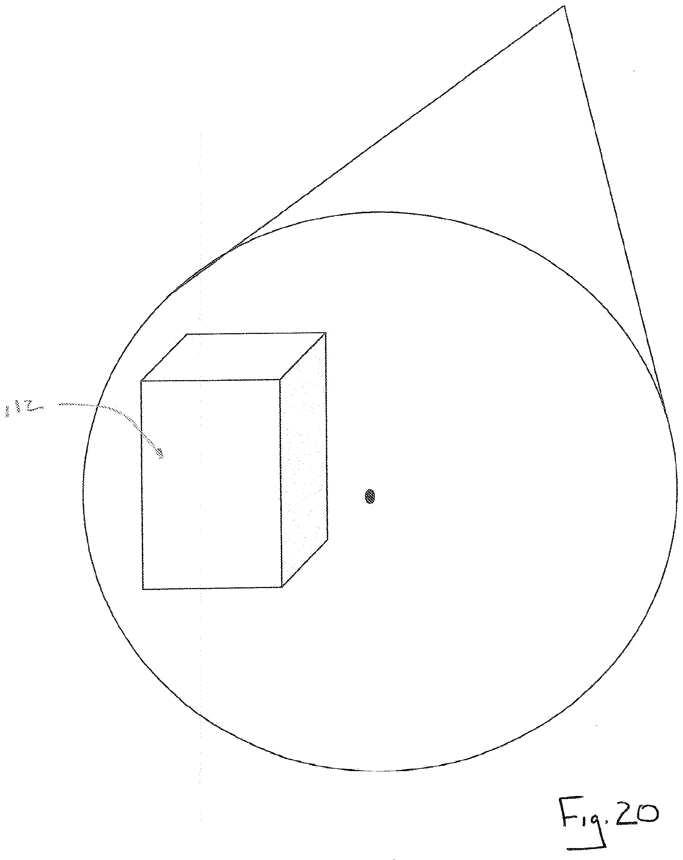

**Fig. 20** depicts a display screen shot illustrating a reconstructed volume displayed over a body diagram.

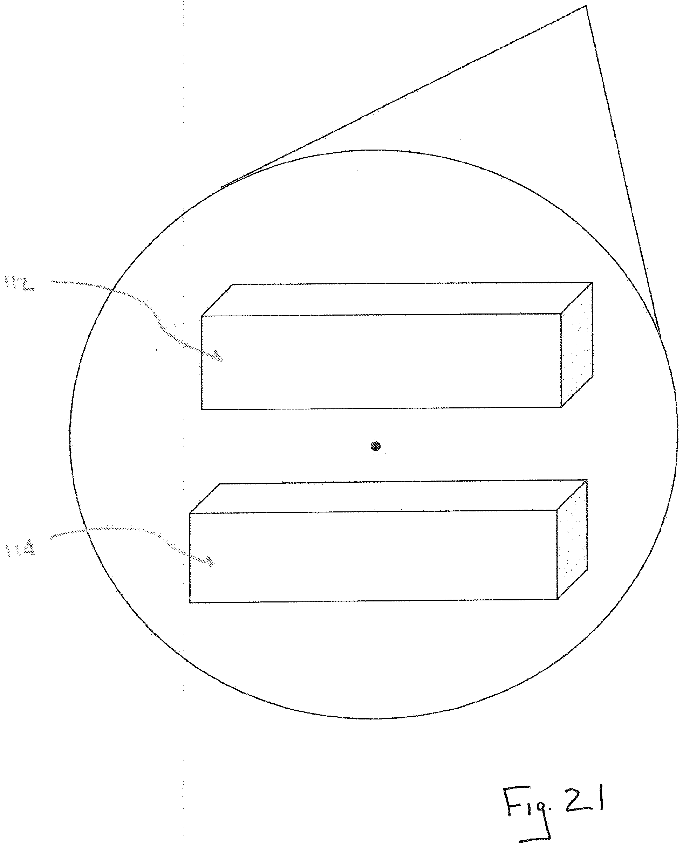

**Fig. 21** depicts a display screen shot illustrating multiple reconstructed volumes displayed over a body diagram.

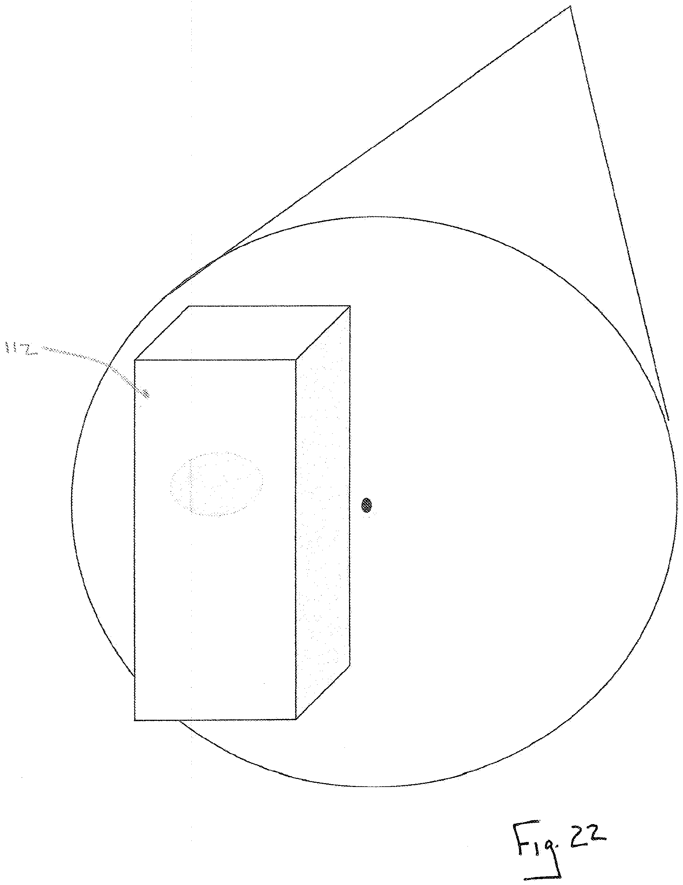

**Fig. 22** depicts a display screen shot illustrating a reconstructed volume of a video clip with the reconstructed volume of an internal lesion, displayed over a body diagram.

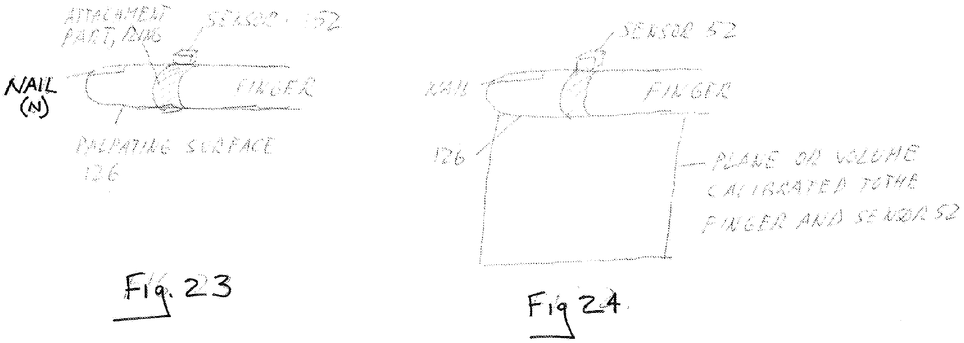

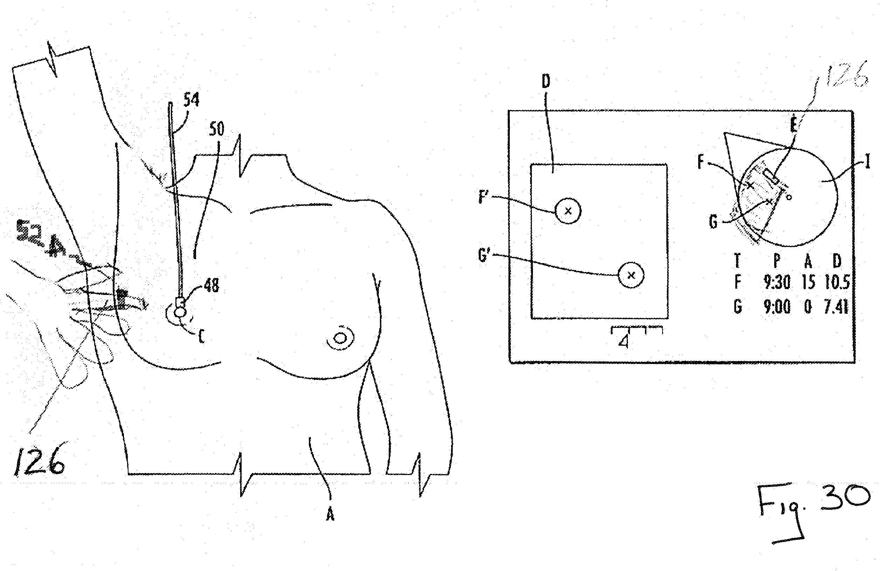

**Fig. 23** illustrates a side view of a finger with sensor 52 for palpation correlation with ultrasound images.
**Fig. 24** illustrates a side view of a finger with sensor 52 and calibrated plane for palpation correlation with ultrasound images with ultrasound display frame.
**Fig**.**30** describes a breast ultrasound exam with palpation correlated with the display of ultrasound images from a previous exam.
**Fig**. **60** is an example of the display of multiple ultrasound probe and frames positions over a body diagram.

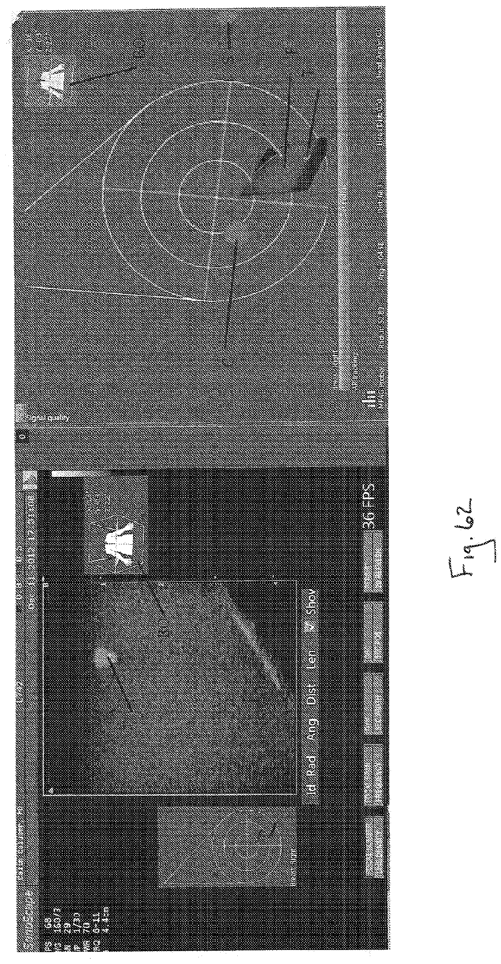

**Fig. 62** is an example of ultrasound frame displayed over a body diagram aligned with the body planes.

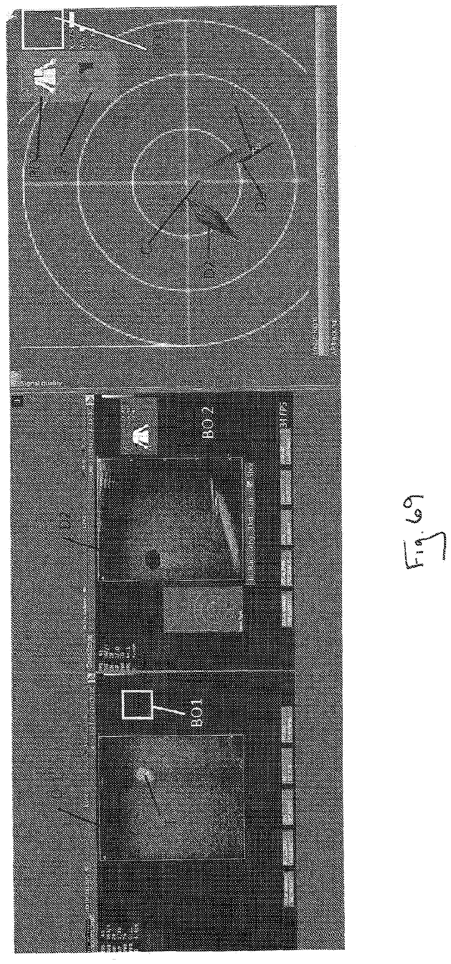

**Fig. 69** is an example of a target relocation screen with multiple guides for probe frame, target and body position/orientation relocation.

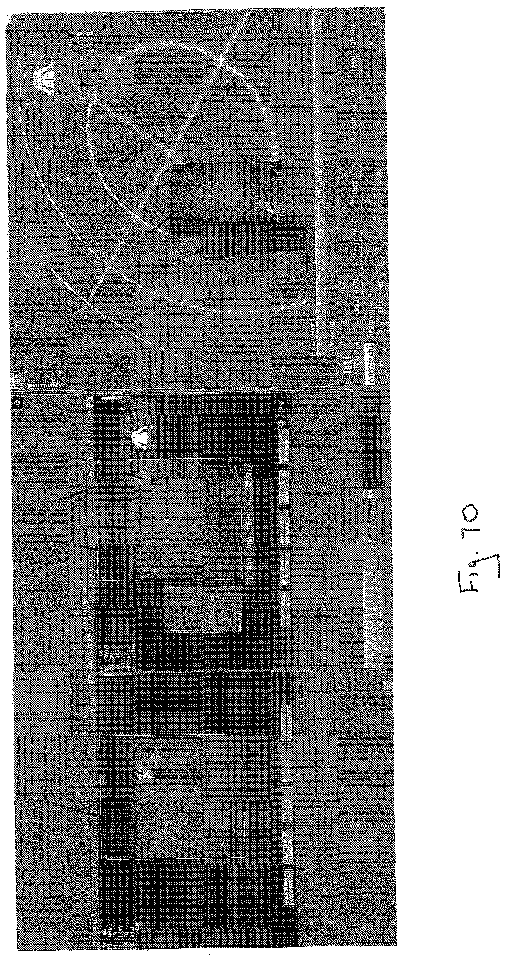

**Fig. 70** is an example of a target relocation screen with multiple guides for probe frame, target and body position/orientation relocation and matched current and previous images.

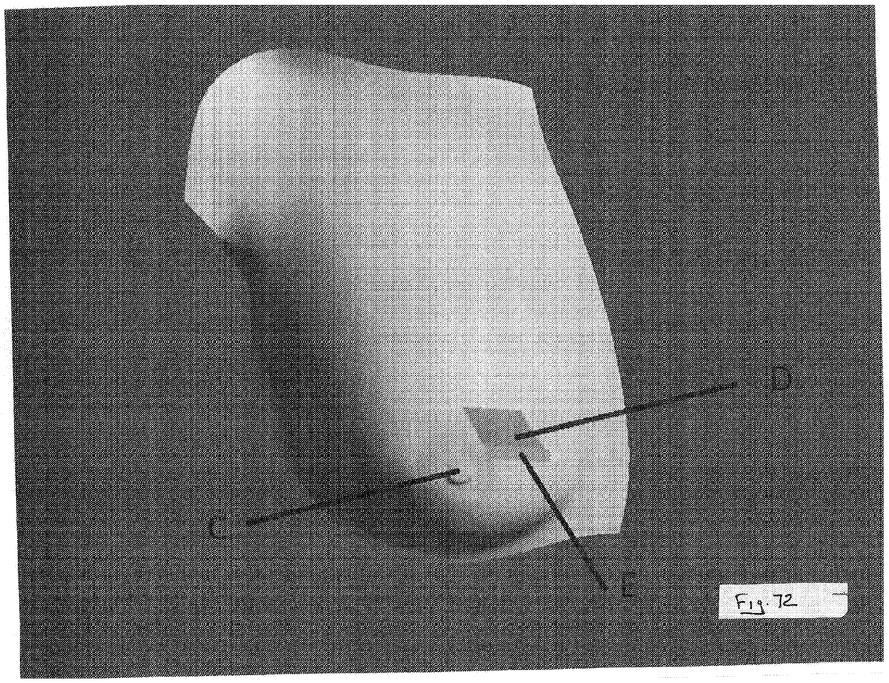

**Fig.72** shows the ultrasound frame displayed over a realistic body diagram.

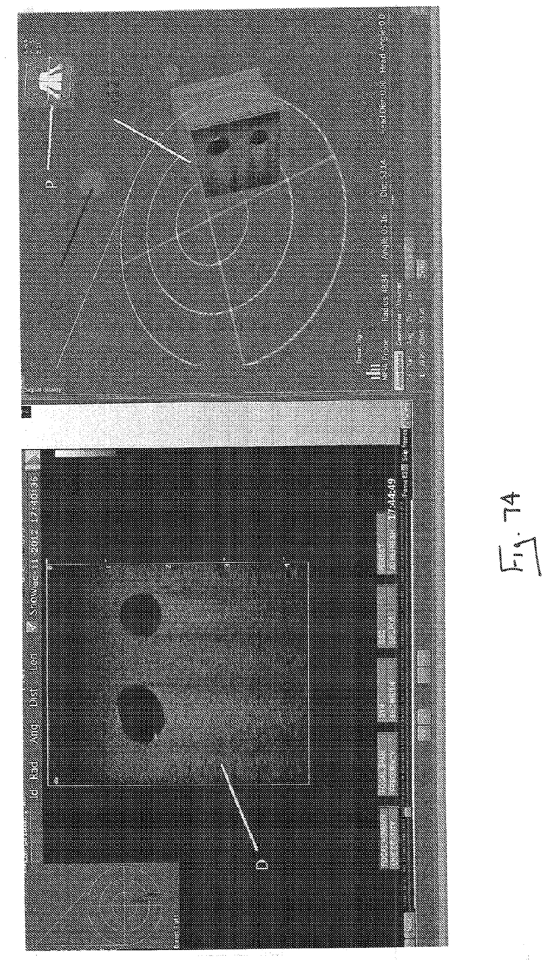

**Fig.74** is an example of an ultrasound video clip with each frame displayed over the body diagram aligned with the patient's orientation planes.

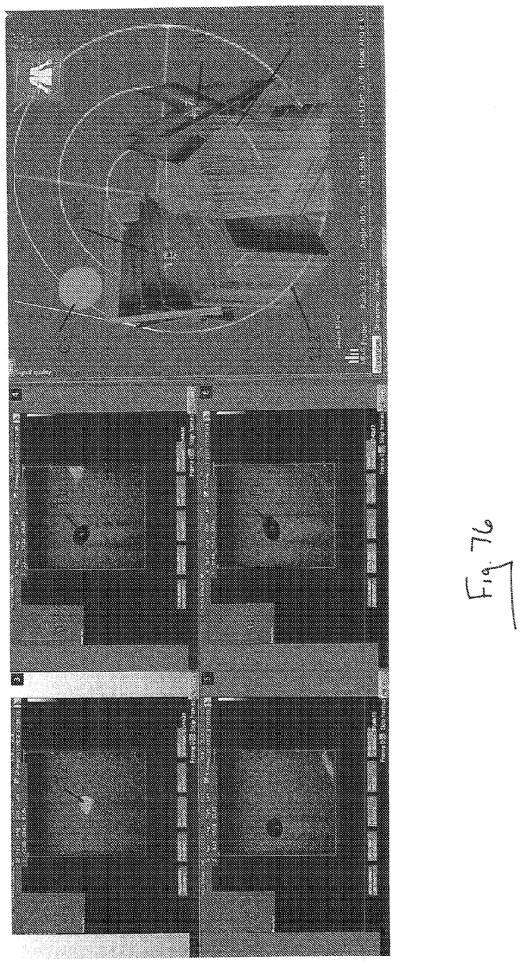

**Fig.76** is an example of multiple ultrasound video clips and individual frames, with each frame displayed over the body diagram aligned with the patient's orientation planes. Target marks and frames corresponding to same lesion are grouped together.

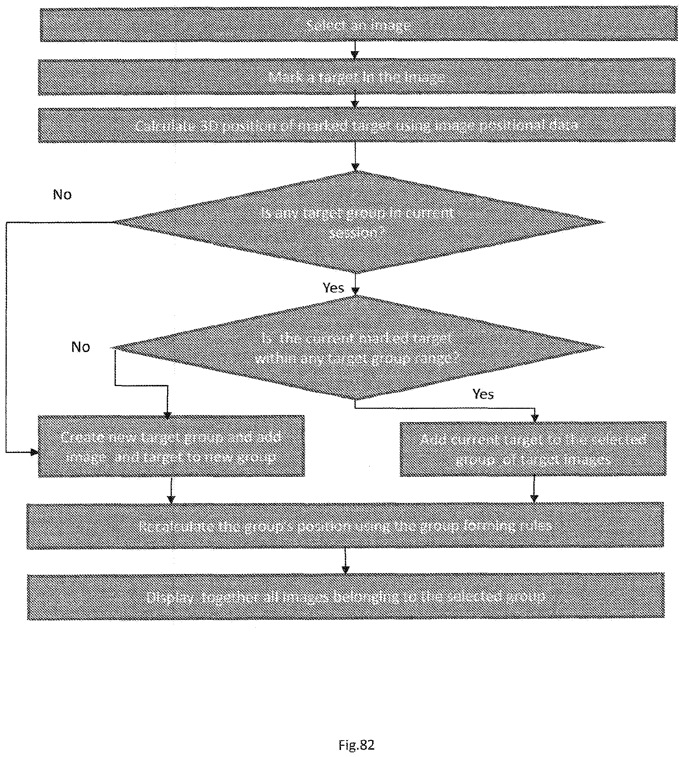

**Fig. 82** shows the steps required to perform the grouping and display of images containing same target.

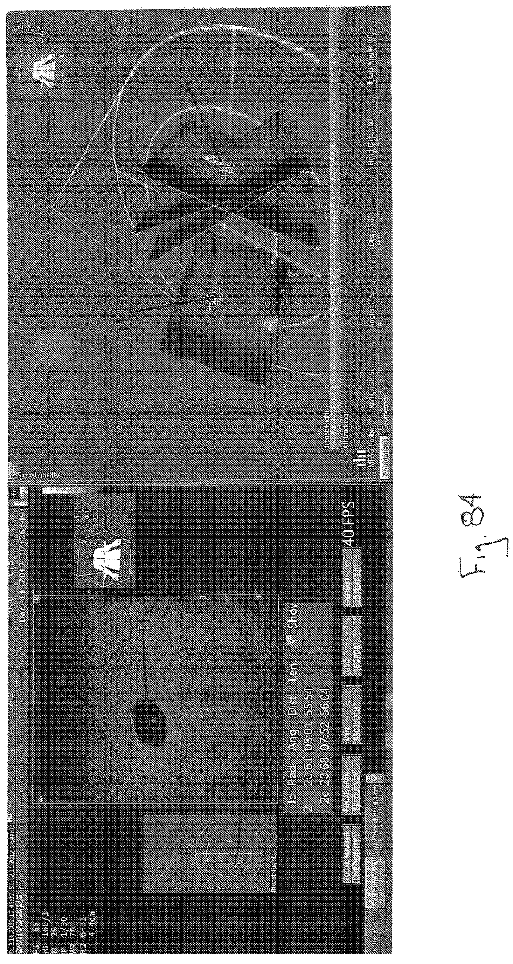

**Fig. 84** is an example of a display with ultrasound images containing same target grouped together over the body diagram.

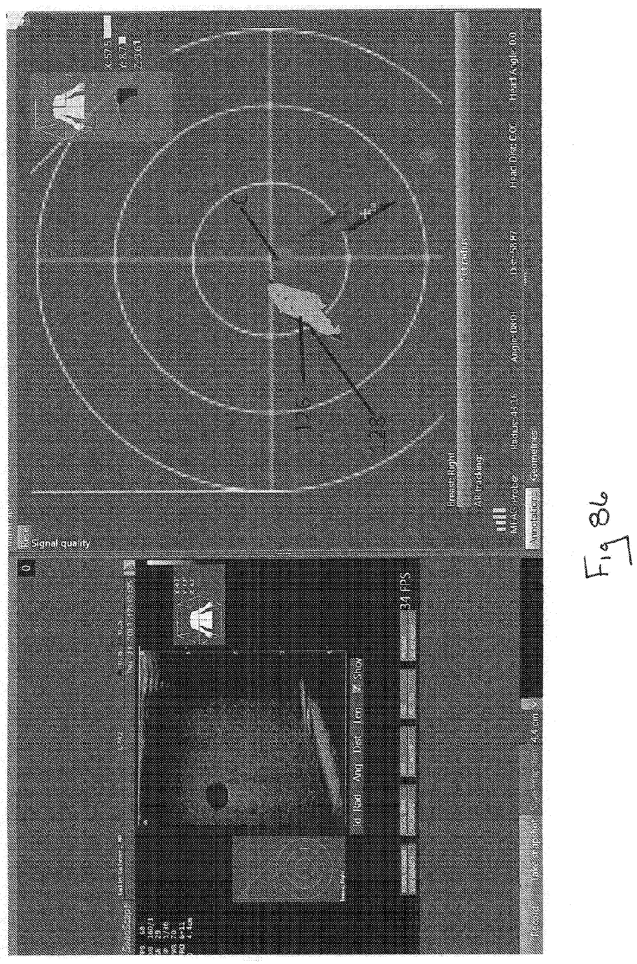

**Fig. 86** is an example of a display showing the palpating finger and ultrasound images over the same body diagram.

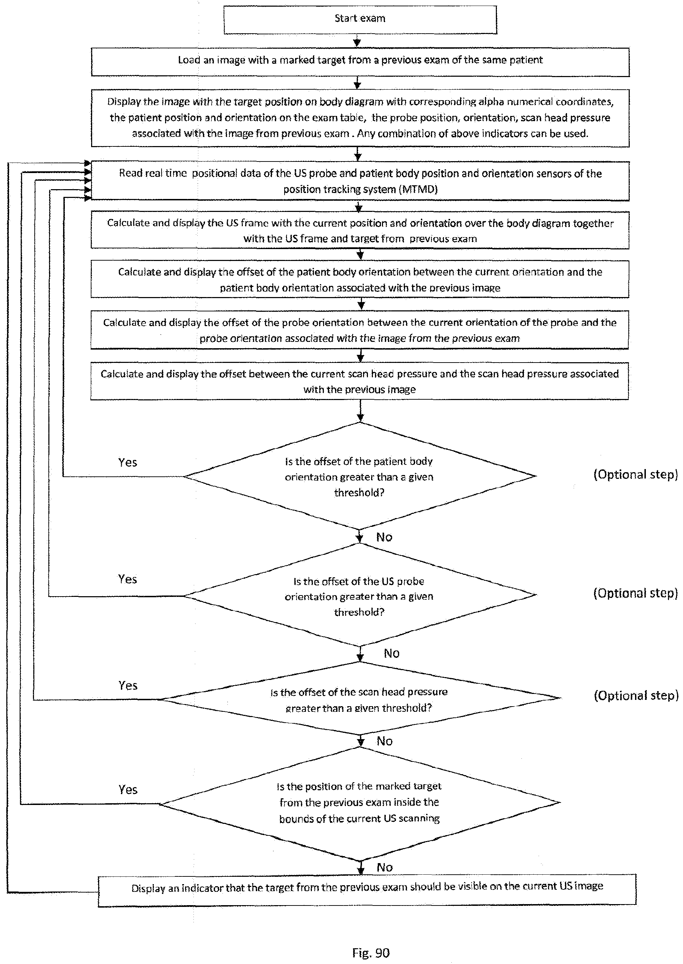

**Fig. 90** shows the steps required to relocate a target from a previous ultrasound exam.

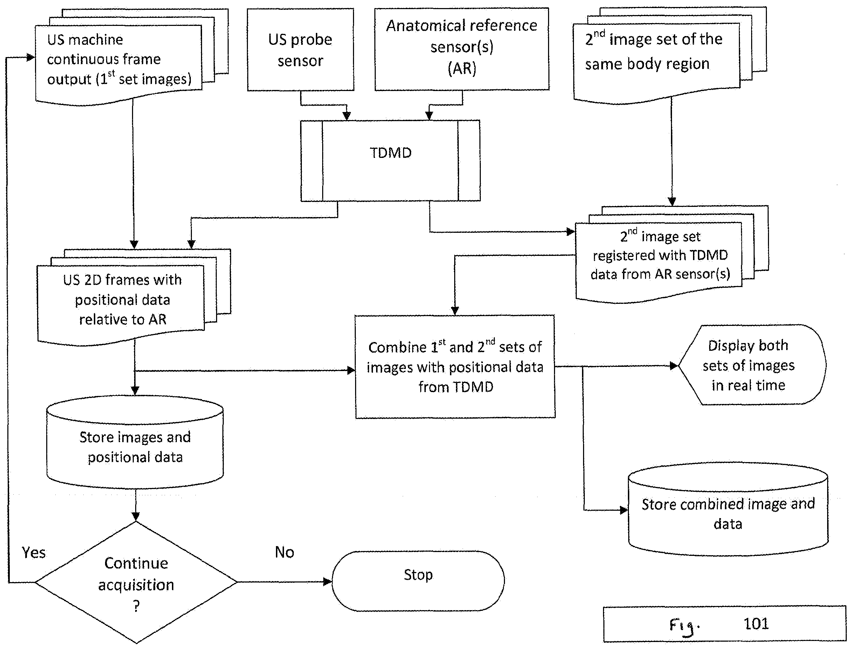

**Fig. 101** shows the steps required to coregister real time ultrasound images with a second set of images of same body region.

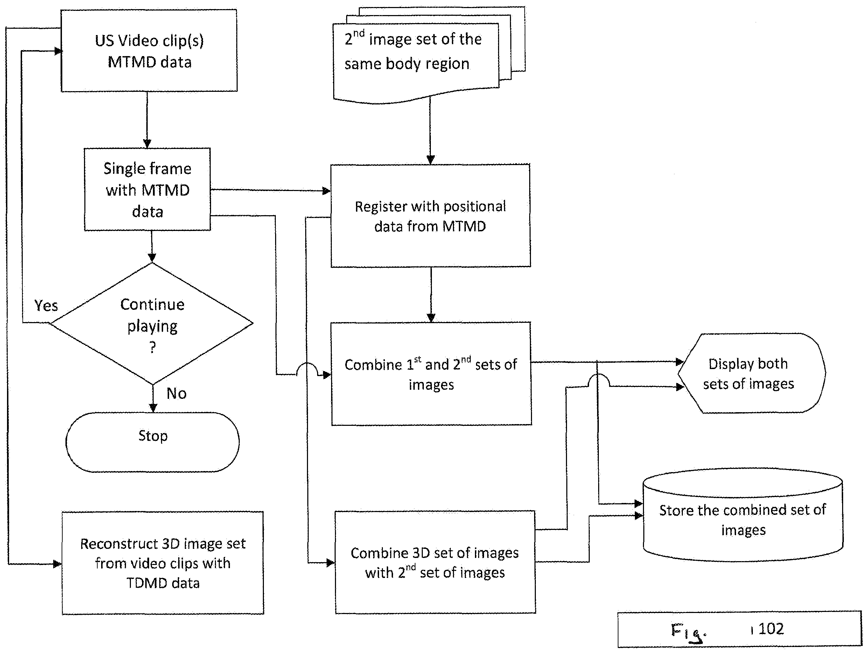

**Fig. 102** shows the steps required to coregister recorded ultrasound images with a second set of images of same body region.

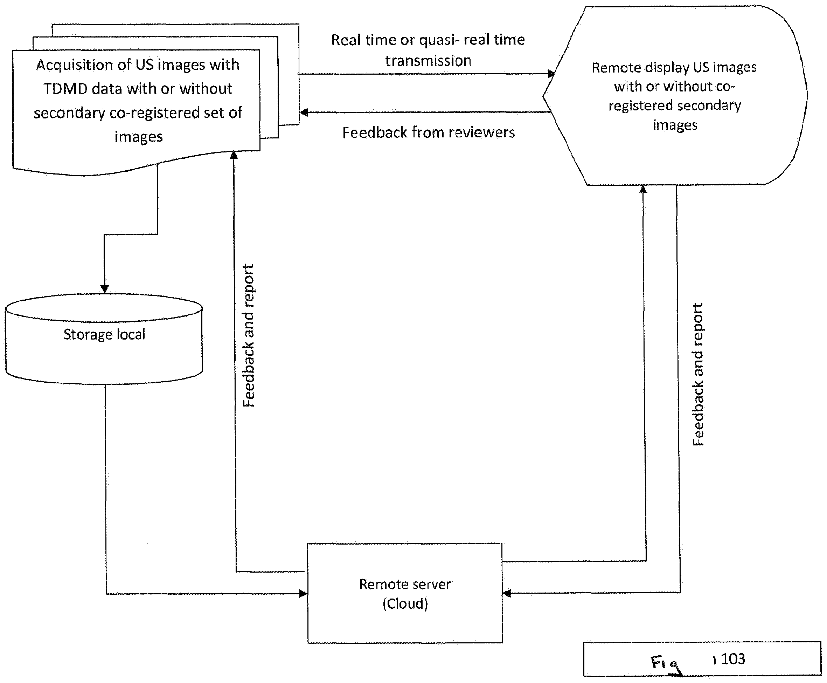

**Fig. 103** with an example for remote interpretation of images acquired with the TDMD.

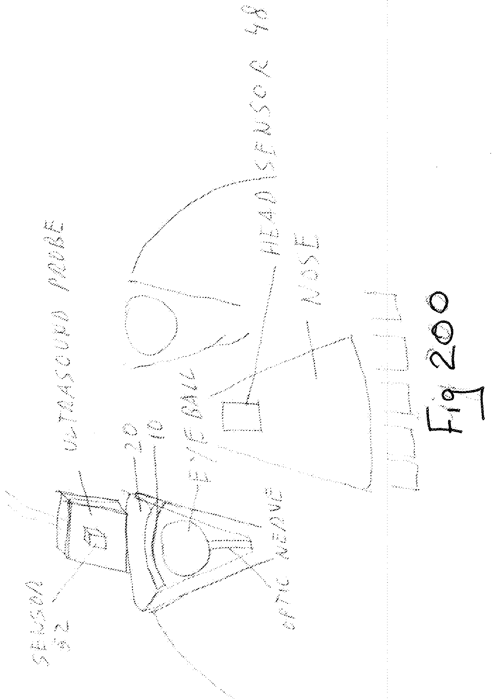

**Fig. 200** shows an ultrasound transducer during eye scanning with the TDMD.

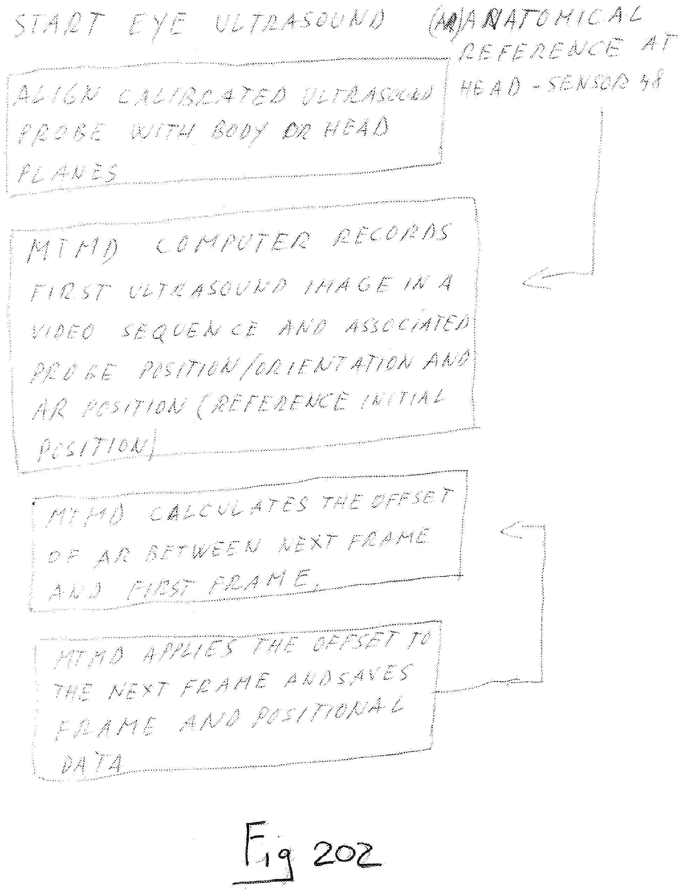

**Fig. 202** shows the steps to acquire images and reconstruct volumes of orbital structures.
**Fig. 210** shows the steps to calculate and display the position of a tracked finger over the body diagram and save positions associated with palpable findings.

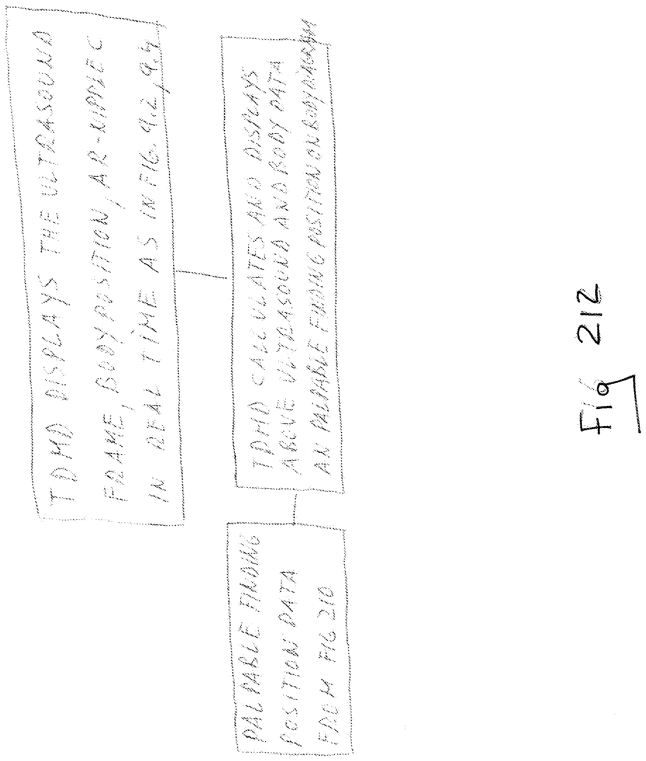

**Fig. 212** shows the steps to calculate and display a palpating finger and previously obtained ultrasound images over a body diagram

### VI. Detailed Description of the Preferred Embodiment

Before explaining the preferred embodiment of the present invention in detail, it is to be understood that the present invention is not limited in its application to the details of arrangements of the components set forth in the following description. As will be appreciated by those skilled in the arts, the present invention is capable of other embodiments and of being practiced and carried out in various ways. Also, it is to be understood that the phraseology and terminology employed herein are for the purpose of description and should not be regarded as limiting. It is also to be understood that where ranges are provided for various aspects of the invention and for examples, they are approximate ranges and are not to be limiting except where noted otherwise.

Unless defined otherwise, all technical and scientific terms used herein have the same meaning as commonly understood by one of ordinary skill in the art to which this invention belongs. Moreover, the singular forms "a", "an", and "the" include plural referents unless the context clearly dictates otherwise. Further, an ultrasound frame as herein described is same with 2D ultrasound image.

It should also be understood that the inventive device may include any of the features and respective hardware components described, or combinations thereof, however some features and components may make more practical sense for one particular use, depending on the particular design considerations and intended use.

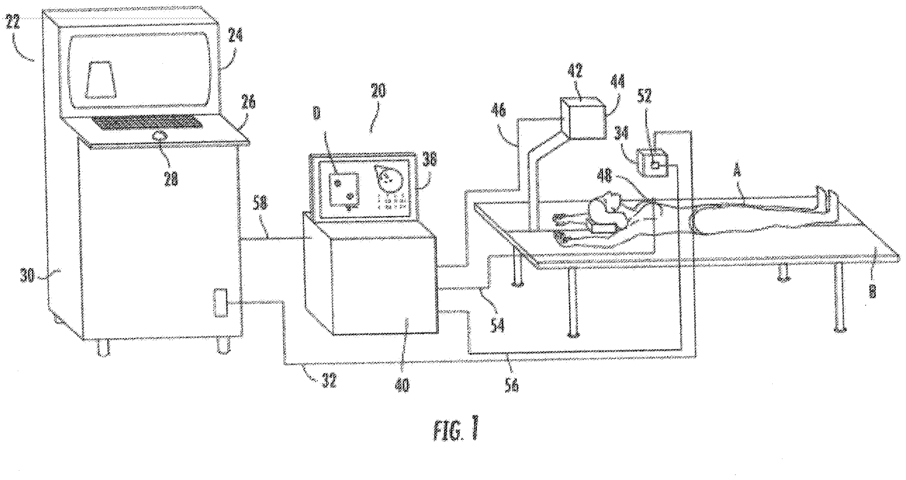

Turning to Figure 1, an over view of the physical aspects of an ultrasound device employing the inventive apparatus 20 is seen. Ultrasound machine 22 is a standard device including display 24, interface with keyboard 26 and pointer 28, chassis containing operating hardware (not seen) 30, probe connecting cord 32, and probe 34.

Inventive apparatus (also referred to as three dimensional mapping display, or TDMD) 20 is depicted and comprises TDMD display 38, TDMD Chassis 40 containing hardware (also referred to as a "processor") and software (not seen; described in detail below), 3D magnetic tracking member 42 with the transmitter 44 connected to TDMD 20 by 3D magnetic tracking member cord 46, first magnetic sensor 48 connected to TDMD 20 by first magnetic, sensor cord 54 and second magnetic sensor 52 connected to TDMD 20 by second magnetic sensor cord 56. A 3^{rd} position sternum sensor, 49 can be attached to track the patient's body position in reference to the exam table, Fig.7. The sensors may also be of a wireless variety, thus sensor cords 56, 58 would not be required. Also a combination of wired and wireless position sensors can be used to provide the position tracking module with positional information from the tracked anatomical references and the ultrasound probe or probes. The positional sensors are used to dynamically track the ultrasound probe and patient's body landmarks that include selected anatomical references and other body locations and provide the data that can be used to coregister the ultrasound real time images with the body diagram or other secondary sets of images, to provide realistic position and orientation information about the ultrasound probe, images and the examined body region (Fig. 9, 502). As those skilled in the arts will understand, the principles of the present invention enable the use of a single display monitor or multiple display monitors for procedures. (For completeness in explaining Figures 1 and 7, patient A is situated on examining table B.)

Turning to Figure 2, a block diagram illustrating the various general working aspects of inventive device 20 is shown. First magnetic sensor (tracking module) 48 and second magnetic sensor (tracking module) 52 provide the positional information to the TDMD 20 3D position board/module 60. Video output 24 from ultrasound device 22 is digitized by the dedicated TDMD module/board 40. It should be noted that the analog to digital image conversion may not be needed if the ultrasound machine can be interfaced and it can directly provide the digital images to the TDMD 22.

TDMD can continuously track one or several anatomical reference markers or positional body markers, which can increase the overall accuracy of the system. If multiple positional body markers are used, not all of them need to be continuously tracked.

To ensure reproducible and accurate mapping of the ultrasound images, magnetic sensors 48 and body position sensor (tracking module) 49 (Fig. 2.1) should be attached at well-defined and reproducible sites, outside or inside the body, during repeated ultrasound exams. First magnetic sensor 48, second magnetic sensor 52, and body position sensor 49 may be used simultaneously or singularly. It should also be noted that the TDMD could accommodate additional positional sensors as well. As a non-limiting example, in the case of breast ultrasound exam, the magnetic sensors should be attached to the Nipple C in the same position during repeated ultrasound exams. For instance, the center of the Nipple C top surface D can be the point of attachment for the anatomical reference position sensor (Fig. 10). It is desirable to have the magnetic sensor wire 54 outside the region of interest to be scanned. Continuing with the breast ultrasound exam example and with a magnetic sensor at the Nipple C, if magnetic sensor wire 54 is aligned in a direction perpendicular to the patient's coronal plane, the entire breast surface may be available for scanning, without the magnetic sensor wire in the path of the ultrasound probe 34.

To address the above, a sensor attachment device 92 (Fig. 10) may be employed to aid the attachment of a wired or wireless magnetic sensor to the Nipple C. Sensor attaching device 92 can be built as a disposable part or as a reusable part after disinfection.

Other configurations will work as well. For non-limiting example, Figure 3 illustrates an alternate configuration in which second optical or infrared sensor 52 provides the positional information to the TDMD 3D position board/module (not shown). The overhead infrared or optical AR tracking system 43 provides the positional information to the TDMD computer 40. Video output 24 from the ultrasound device 22 is digitized by the dedicated TDMD module/board 40. Again, analog to digital image conversion may not be required if the ultrasound device 22 can be interfaced and directly provide the digital images to TDMD computer 40. The digital ultrasound images with the associated positional information are displayed in the TDMD computer display 38 or stored for review and processing at a later time.

Turning to Figure 4, a block diagram illustrating the various general working aspects of inventive device 20 are shown. Second magnetic sensor 52, which can be of any variety such as optical or infrared provides the positional information to the TDMD 20 3D position board/module 60 and overhead infrared position detector 43 transmits positional information to TDMD computer 40. Video output 24 from ultrasound device 22 is digitized by the dedicated TDMD module/board 40. It should be noted that the analog to digital image conversion may not be needed if the ultrasound machine 22 can be interfaced and it can directly provide the digital images to the TDMD 40.

Returning to Figure 1, second magnetic sensor 52 is attached to the exterior of probe 34 and, as seen in more detail in Figure 5, first magnetic sensor 48 is positioned at the anatomical reference, here, the breast nipple C of Patient A.

Ultrasound device 22 video output 24 is directed to TDMD video capture board at TDMD Chassis 40 through video output cord 58 as is 3D magnetic tracking member 42 through 3D magnetic tracking member cord 46. TDMD display 38 is then enabled to shows images D captured by ultrasound device 22 and associated positional data as collected from 3D tracking member 42, first magnetic sensor 48 and second magnetic sensor 52.

Turning to Figure 5, a detailed view of probe 34 with the second magnetic sensor 52 and first magnetic sensor 48 applied at the upper margin of the right Nipple C. First magnetic sensor 48 continuously tracks the anatomical reference position, the Nipple C in this case, to compensate for motion registration errors during the ultrasound exam. Figure 6 illustrates TDMD display 38 with the captured video image D from the ultrasound machine and the body diagram of Figure 5 with the probe 34 position and orientation at the time of image capture D and two different targets F and G in body part diagram I, and F' and G' as selected in image D image capture.

Additionally, each target is displayed with the associated position (clock face position with hourly representation or degrees to longitudinal axis and anatomical reference as center) and distance (cm) from the selected anatomical reference. Positional coordinates are displayed under body part diagram I in Figure 6. While the inventive device can enable any number of coordinates to be displayed, here the example includes Target number (T), example F and G, Position in reference to anatomical reference in hourly format (here, 9:30 for F and 9:00 for G), position from anatomical reference in degrees (here, 15° for F and 0° for G), and distance from anatomical reference in centimeters (cm) (here, 10.5cam for F and 7.41cm for G). When anatomical reference sensors 48 and 49 are used to dynamically track the position of the nipple and patient's body, the clock face position can be calculated in reference to the real time patient's body orientation planes, which would increase the accuracy and reproducibility of measured targets positional coordinates Fig (2.1, 9.1). Also, probe 34 is identified at transducer position Icon E for its position location and orientation.

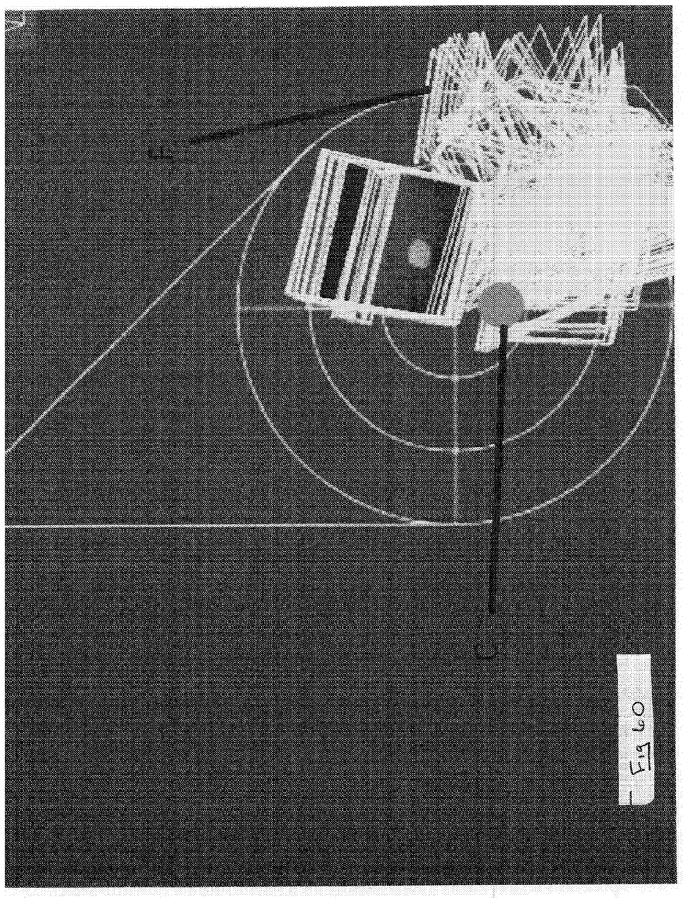

An additional function is to display a cumulative area of the transducer positions (via icon E) over the body diagram, where the ultrasound images of breast tissue were generated and displayed in real time, during patient examination. Fig.60 displays all ultrasound frames generated while scanning the breast (F) over the breast diagram with nipple C. This will allow for a quick evaluation of ultrasound examination completeness and demonstrate the region evaluated by the operator. The display of cumulated frames position and orientations can be done at the time of the examination or at a later time. A more detailed description is following in this patent application.

In the preferred embodiment, any off the shelf generic PC computer with Windows XP®, Windows 7 (by Microsoft Corporation, Redmond, WA) can be used to run instructions compiled in C++ and dotnet languages. While preferred, those skilled in the arts will understand that the invention can be implemented on any other computer platform and operating system.

The software substantially used to process the data received by the processor from the at least one sensor and data from the ultrasound to manipulate the data for identifying, and storing in memory as selected by the user, target site location and size information in relation to selected anatomical reference(s) for simultaneous review and interpretation and later retrieval for comparative purposes with later examination, whether compared in real time or a later time based upon saved data. The inventive device enabling a user to accurately review, evaluate, and compare examination results by having anatomical reference(s) guides to isolate target sites.

The body diagram representation is not limited to the "bird's eye view" type like the "clock" representation for the breast, but more complex and realistic three dimensional representations of the body or body regions, including images obtained with other modalities like MRI, mammograms, gamma cameras or positron emission tomography and using contour rendering algorithms, can be used. The calculated and recorded positional data can be displayed in these representations. The ultrasound transducer position, orientation, can be depicted in a realistic appearance in space so it can be easily reproduced at subsequent examinations.

Additionally, the preferred 3D position registration system is based on magnetic tracking technology (for example, like that manufactured by Ascension Technology, Burlington, VT); however, any other suitable technology, such as optical or ultrasound, may be employed. Moreover, the inventive device can be deployed as an add-on to any existing ultrasound unit, and can outfit DICOM compatible and non-DICOM machines as well. The infrared sensors, also commercially available (Natural Point Inc., Corvallis, OR), comprise at least one infrared camera with the dedicated hardware and software receiving reflected infrared light from the reflectors or emitted infrared light from small infrared light sources applied over the anatomical references. The infrared cameras can be replaced with optical cameras and the infrared reflectors or emitters with optical markers or light emitters. One or more infrared or optical cameras can also be used.

The ultrasound probe and anatomical reference point real time tracking is not limited to the above solution, but other tracking modalities like ultrasound, optical, inertial etc. can be used for the ultrasound probe and optical/pattern recognition, magnetic, etc. for the anatomical reference point real time tracking. It should also be noted that tracking modalities can be used in combination with one another, for non-limiting example, ultrasound tracking with optical tracking. It is also notable that the described TDMD system and method can optionally be used with the anatomical reference tracking feature disabled.

In any of the above configurations, initial calibration is needed to register the ultrasound probe scanning plane orientation and position. Any 3D calibration method for 2D ultrasound probes as available in the published literature can be used.

The position of a small tumor or other target in the breast, or other body part, depends on the patient's body position due to the gravity effect, ultrasound probe position and orientation which can displace the tissue under the probe and the pressure applied by the operator on the probe. To obtain accurate reproducible positional coordinates of a lesion, the above conditions need to be measured and able to reproduce at a subsequent exam.

Turning to Figure 9, the TDMD operation steps required to record the 3D position of targets in relation to anatomical references are shown. For each patient, at the beginning of examination the anatomical reference spatial position, patient's body position and the ultrasound probe position relative to anatomical reference(s) and its orientation relative to the body anatomical planes are defined in a spatial coordinate system and recorded, (Fig. 9, 501). This step provides the reference for the coregistration of the ultrasound probe and images with the body diagram or secondary set of body images. One method is to hold the ultrasound probe scan-head center at the anatomical reference, for example, on the Nipple C, with the probe 34, fitted with position sensor 52, held in a known orientation with the patient's body planes and axes, for example sagittal plane, horizontal, parallel to the patient and examination table long axis (Fig 1) to determine the patient's position and orientation axes and planes. In this step the nipple C position is set with the position coordinates at the center of the probe and the known patient's plane, sagittal for example, is set using the coordinates of the probe scan plane. This method does not provide dynamic positional tracking for nipple C and patient's orientation planes, therefore patient motion will likely lead to position registration errors in the ultrasound images. At least one anatomical reference needs to be defined at the beginning of each examination, however more than one anatomical references can be defined, which can increase the measurements accuracy. During the anatomical reference setting step and during scanning a second magnetic sensor 52 is used to track the ultrasound probe, the first magnetic sensor 48 position attached at the anatomical reference (nipple C) is recorded and computed by the TDMD 40, so it can continuously track the anatomical reference. In this configuration with 2 sensors, (52 and 48), the nipple C position coordinates are obtained from sensor 48 and only the patient's body planes needs to be set, for example by holding the probe with the scan plane parallel with a known patient's plane and set the patient orientation planes (Fig 9.3). This method provides the dynamic referencing of the nipple C or other monitored anatomical reference but is limited due to the static referencing of patient's orientation planes. A method where the patient's body orientation planes are dynamically referenced is described below. An additional calculation provides the offset between the anatomical reference point and first sensor 48 position and is necessary when the first position sensor 48 is applied in close proximity, but slightly off the selected anatomical reference. This is a non-limiting method to measure and apply the positional offset between sensor 48 and an anatomical reference point. In other embodiments with wireless anatomical reference sensors or markers, for example when using the overhead anatomical reference tracking system with infrared or optical sensors or markers, are applied exactly at the anatomical reference point, this additional correction is not necessary and can be omitted. If a wired anatomical reference marker can be applied exactly at the anatomical reference point, this additional correction is not necessary and can be omitted.

During an ultrasound exam, the patient's body position and orientation can change, which can have an effect on the measurement and description of a lesion's position. During the real time ultrasound exam image acquisition and capture, each internal ultrasound target position relative to the anatomical references depends, among other factors, on the patient's position relative to the direction of the gravity force or the earth's magnetic field. Therefore the positional relation between the patient's body position and an examination table, B or other reproducible fixed reference used to position the patient, a chair or a wall for example, can be associated with the ultrasound images or other images of the body, to aid repositioning the patient at subsequent imaging and match the gravity force effect between temporally distinct image sets. The gravity force effect is larger on deformable structures, like the breast. For example, during a breast ultrasound exam, the position of a small target in the breast relative to the nipple or other anatomical reference can change between the supine and half decubitus patient positions on the examination table. Unlike the approaches of the prior art, at the follow up exams or during the same patient exam, the patient whole body position can be adjusted to match the body position relative to the examination table or other known fixed reference object recorded with the previously obtained ultrasound images and help finding a target with the previously recorded coordinates relative to the same anatomical references.

The examination table, B, or other reference object with known position is registered in the same spatial reference frame with the ultrasound probe and images and patient's body position. The first set of images which can be the ultrasound images are coregistered with the second set of images which can be the patient's body diagram and which can be coregistered with a third set of images which are of the examination table, B, or other object with a reproducible position related to the gravity force direction. The coregistration of the three sets of images can be performed dynamically for all image sets, with positional sensors or markers providing continuous or quasi continuous output or a combination of dynamic and static registration, where one or more anatomical references are dynamically tracked at the body and the examination table or third set of images position can be tracked with dynamic sensors or spot measurements if fixed in the spatial reference frame during the ultrasound exam, like an examination table (Fig. 9.4) The examination table B position can be determined and saved for future use in the spatial frame coordinates, if permanently aligned with the spatial frame or can be dynamically determined with a position sensor attached to the table if the table moves in the spatial frame. Examples of compatible spatial reference frames include magnetic, optical, infrared, ultrasound or combinations of two or more types of positional reference frames with the corresponding transmitters and sensors or markers. The coregistered sets of images can be displayed together or separately, temporally synchronized or not synchronized. Also, it is possible to display at same time coregistered sets of images of same body region obtained at different times, to facilitate the image comparison process for diagnostic and treatment purposes.

The patient's whole body position and orientation representation, BO, examination table, or other fixed reference object position and orientation, B, position and coordinates of Nipple, C, position and coordinates of a target, T, position and orientation of the ultrasound image, E, position of sensor 49 on body, S, can be recorded with each 2D ultrasound frame Fig 62. The above positional representations and corresponding alpha numerical values can be displayed and recorded in any combination or order.

There are multiple methods to align the patient's body planes and axes with position sensors and an exam table or other objects.

A preferred embodiment describes a nipple sensor for nipple position tracking 48, and a body position sensor which can be attached to the sternum or other body part 49 and connected with above described apparatus and system.

The patient's whole body position recording can be automated in the TDMD by tracking and recording the position coordinates of the anatomical reference sensor or sensors attached to the patient's body and compared with a reference body position coordinates.

In one embodiment, with the patient's body in the supine or other known reproducible body position on an exam table, B, body position sensor 49 attached to the patient, the output from the position sensor 49 can be used to measure and set the body reference position and orientation in the TDMD, associated with the known patient position on table. The patient's reference body planes or axes can be set to match the known exam table axes or any other known orientation planes, including the probe scan plane when aligned with one or more of the patient's body planes and axes (Figs. 9.2, 9.6). After setting the patient's body reference planes in the spatial frame, the output from body sensor 49 can measure changes in the body position and orientation during the imaging session. The patient's body position can be represented as the 3 orthogonal

## Claims

1. A medical ultrasound system for reproducing the location of an ultrasound probe and image in relation to a pre-selected anatomical reference of a patient, the ultrasound system including:
an ultrasound machine (22) comprising said probe having a probe head,
an ultrasound image generator for creating image data and said image, and a real time three-dimensional image position image processor (20)) comprising:
a probe sensor (52) fixed to the probe head for tracking probe head position;
an anatomical reference sensor (48) for tracking the position of the pre-selected anatomical reference;
a body sensor (49) for tracking the patient's body position and orientation in reference to a known patient position on a table;
a processor configured to receive tracking data from the probe sensor, the anatomical reference sensor, and the body position sensor, and the image from the ultrasound image generator, the processor dynamically referencing probe head position, and the image to the anatomical reference and the body or a second prior set of images and, further, automatically generating annotations of image targets selected by an operator;
a display for simultaneously displaying the image and the probe head position over a body diagram dynamically referenced to the pre-selected anatomical reference and the patient's body orientation planes;
memory for storing data from the processor for later retrieval and overlay with subsequent images to facilitate anatomical reference correlation and location of image targets.

2. The system of Claim 1, wherein the display (38) is further configured for simultaneously displaying the probe (34) orientation over the body diagram.

3. The system of Claim 1 - 2, wherein the data stored in the memory allows later retrieval and overlay with subsequent ultrasound data to facilitate anatomical reference correlation and target site localization.

4. The system according to any one of the preceding claims, wherein the anatomical reference sensor is attachable by means of an attachment device (92).

5. Method for reproducing the location of an ultrasound probe and image in relation to a pre-selected anatomical reference of a patient with a system according to claim 1 - 4, comprising the following steps:
- pre-selecting an anatomical reference;
- receiving, by the processor, tracking data from the probe sensor (52), the anatomical reference sensor (48), and the body position sensor, and the image from the ultrasound image generator;
- dynamically referencing, by the processor, probe head position, and the image to the anatomical references and the body or a second prior set of images and, further, automatically generating annotations of image targets selected by an operator;
- simultaneously displaying on the display the image and the probe head position over a body diagram dynamically referenced to the pre-selected anatomical reference and the patient's body orientation planes;
- storing data from the processor for later retrieval and overlay with subsequent images to facilitate anatomical reference correlation and location of image targets.

6. Method according to claim 5 comprising the further step of simultaneously displaying the probe (34) orientation over the body diagram.

7. Method according to the preceding claim 5 comprising the further step of displaying at the same time coregistered sets of images of the same body region obtained at different times.

## Patentansprüche

1. Medizinisches Ultraschallsystem zum Reproduzieren des Ortes einer Ultraschallsonde und Bildes in Bezug auf eine vorausgewählte anatomische Referenz eines Patienten, wobei das Ultraschallsystem umfasst:
ein Ultraschallgerät (22), aufweisend die Sonde mit einem Sondenkopf, einem Ultraschall-Bildgenerator zum Erzeugen von Bilddaten und des Bildes,
und einen dreidimensionalen Echtzeit-Bildprozessor (20) der Bildposition, aufweisend: einen Sondensensor (52), welcher an dem Sondenkopf befestigt ist, um die Sondenkopfposition zu verfolgen; einen anatomischen Referenzsensor (48), um die Position der vorausgewählten anatomischen Referenz zu verfolgen;
einen Körpersensor (49), um die Körperposition und Orientierung des Patienten in Bezug auf eine bekannte Patientenposition auf einem Tisch zu verfolgen;
einen Prozessor, welcher dafür ausgelegt ist, Verfolgungsdaten von dem Sondensensor, dem anatomischen Referenzsensor und dem Körperpositionssensor und das Bild von dem Ultraschall-Bildgenerator zu empfangen, wobei der Prozessor die Sondenkopfposition und das Bild zu der anatomischen Referenz und dem Körper oder einem zweiten früheren Satz von Bildern dynamisch referenziert und weiter automatisch Anmerkungen von durch eine Bedienperson ausgewählten Bildzielen erzeugt;
einen Bildschirm, um gleichzeitig das Bild und die Sondenkopfposition über einem zu der vorausgewählten anatomischen Referenz dynamisch referenzierten Körperdiagramm und den Körperorientierungsebenen des Patienten anzuzeigen;
Speicher zum Abspeichern von Daten von dem Prozessor für spätere Abfrage und Überlagerung mit nachfolgenden Bildern, um anatomische Referenzkorrelation und -ort von Bildzielen zu erleichtern.

2. System nach Anspruch 1, wobei der Bildschirm (38) weiter dafür ausgelegt ist, um gleichzeitig die Orientierung der Sonde (34) über dem Körperdiagramm anzuzeigen.

3. System nach Anspruch 1 - 2, wobei die in dem Speicher abgespeicherten Daten späteren Abruf und Überlagerung mit nachfolgenden Ultraschalldaten ermöglichen, um anatomische Referenzkorrelation und Standortbestimmung des Zielortes zu erleichtern.

4. System nach einem der vorhergehenden Ansprüche, wobei der anatomische Referenzsensor mittels einer Befestigungsvorrichtung (92) befestigbar ist.

5. Verfahren zum Reproduzieren des Ortes einer Ultraschallsonde und Bildes in Bezug auf eine vorausgewählte anatomische Referenz eines Patienten mit einem System nach den Ansprüchen 1 - 4, aufweisend die folgenden Schritte:
- Vorauswählen einer anatomischen Referenz;
- Empfangen, durch den Prozessor, von Verfolgungsdaten von dem Sondensensor (52), dem anatomischen Referenzsensor (48) und dem Körperpositionssensor und des Bildes von dem Ultraschall-Bildgenerator;
- dynamisches Referenzieren, durch den Prozessor, von Sondenkopfposition und dem Bild zu den anatomischen Referenzen und dem Körper oder einem zweiten früheren Satz von Bildern und weiter automatisches Erzeugen von Anmerkungen von durch die Bedienperson ausgewählten Bildzielen;
- gleichzeitiges Anzeigen des Bildes und der Sondenkopfposition über einem zu der vorausgewählten anatomischen Referenz und den Körperorientierungsebenen des Patienten dynamisch referenzierten Körperdiagramm;
- Abspeichern von Daten von dem Prozessor für spätere Abfrage und Überlagerung mit nachfolgenden Bildern, um anatomische Referenzkorrelation und -ort von Bildzielen zu erleichtern.

6. Verfahren nach Anspruch 5, aufweisend den weiteren Schritt des gleichzeitigen Anzeigens der Orientierung der Sonde (34) über dem Körperdiagramm.

7. Verfahren nach dem vorhergehenden Anspruch 5, aufweisend den weiteren Schritt des Anzeigens zur gleichen Zeit von koregistrierten Sätzen von Bildern der gleichen Körperregion, welche zu verschiedenen Zeiten erzielt wurden.

## Revendications

1. Un système ultrasonore médical destiné à reproduire la localisation d'une sonde ultrasonore et d'une image par rapport à une référence anatomique présélectionnée d'un patient, le système ultrasonore comprenant :
un échographe (22) comprenant ladite sonde dotée d'une tête de sonde,
un générateur d'image ultrasonore pour créer des données d'image et ladite image,
et un processeur de position d'image tridimensionnelle en temps réel (20) comprenant :
un capteur de sonde (52) fixé sur la tête de sonde pour suivre la position de la tête de sonde ;
un capteur de référence anatomique (48) pour suivre la position de la référence anatomique présélectionnée ;
un capteur de corps (49) pour suivre la position et l'orientation du corps du patient par rapport à une position connue du patient sur une table ;
un processeur configuré pour recevoir les données de suivi du capteur de sonde, du capteur de référence anatomique et du capteur de corps, et l'image du générateur d'image ultrasonore, le processeur référençant dynamiquement la position de la têt de sonde, et l'image par rapport à la référence anatomique et au corps, ou une deuxième série de données antérieures, puis générant automatiquement des annotations de cibles d'image sélectionnées par un opérateur ;
un écran pour afficher simultanément l'image et la position de la tête de sonde sur un diagramme de corps référencé dynamiquement par rapport à la référence anatomique présélectionnée et des plans d'orientation du corps du patient ;
une mémoire pour stocker les données du processeur pour une récupération ultérieure et une superposition d'images ultérieures et faciliter la corrélation entre référence anatomique et localisation des cibles d'image.

2. Le système selon la Revendication 1 étant précisé que l'écran (38) est en outre configuré pour afficher simultanément l'orientation de la sonde (34) sur le diagramme du corps.

3. Le système selon la Revendications 1 et 2, étant précisé que les données stockées dans la mémoire permettent une récupération ultérieure et une superposition avec des données ultrasonores ultérieures afin de faciliter la corrélation entre référence anatomique et localisation du site cible.

4. Le système selon l'une quelconque des Revendication précédentes, étant précisé que le capteur de référence anatomique peut être attaché au moyen d'un dispositif de fixation (92).

5. Procédé pour reproduire la localisation d'une sonde ultrasonore et d'une image par rapport à une référence anatomique présélectionnée d'un patient avec un système conformément aux Revendications 1 à 4, comprenant les étapes suivantes :
- présélection d'une référence anatomique ;
- réception, par le processeur, de données de suivi du capteur de sonde (52), du capteur de référence anatomique (45) et du capteur de position du corps, et de l'image du générateur d'image ultrasonore ;
- référencement dynamique, par le processeur, de la position de tête de capteur, et de l'image par rapport aux références anatomiques et au corps, ou d'une deuxième série de données antérieures, puis génération automatique des annotations de cibles d'image sélectionnées par un opérateur ;
- affichage simultané sur l'écran de l'image et de la position de la tête de sonde sur un diagramme de corps référencé dynamiquement par rapport à la référence anatomique présélectionnée et des plans d'orientation du corps du patient ;
- stockage des données du processeur pour une récupération ultérieure et une superposition d'images ultérieures pour faciliter la corrélation entre référence anatomique et localisation des cibles d'image.

6. Procédé selon la Revendication 5 comprenant en outre l'étape d'affichage simultané de l'orientation de la sonde (34) sur le diagramme du corps.

7. Procédé selon la Revendication 5 précédente comprenant en outre l'étape d'affichage simultané de séries d'images coréférencées de la même région du corps obtenues à des moments différents.
